(19) Europäisches Patentamt / European Patent Office / Office européen des brevets

(11) **EP 3 884 873 B1**

(12) **EUROPEAN PATENT SPECIFICATION**

(45) Date of publication and mention
of the grant of the patent:
**28.12.2022  Bulletin 2022/52**

(21) Application number: **19903084.2**

(22) Date of filing: **11.09.2019**

(51) International Patent Classification (IPC):
$A61B\ 10/00$ (2006.01)   $A61B\ 3/113$ (2006.01)
$A61B\ 3/00$ (2006.01)   $A61B\ 3/032$ (2006.01)
$A61B\ 3/15$ (2006.01)   $A61B\ 5/107$ (2006.01)
$A61B\ 5/00$ (2006.01)

(52) Cooperative Patent Classification (CPC):
**A61B 3/152; A61B 3/0033; A61B 3/0041;
A61B 3/032; A61B 3/113; A61B 5/1075;
A61B 5/1079; A61B 5/4088**

(86) International application number:
**PCT/JP2019/035717**

(87) International publication number:
**WO 2020/137023 (02.07.2020 Gazette 2020/27)**

(54) **EVALUATION DEVICE, EVALUATION METHOD, AND EVALUATION PROGRAM**

BEURTEILUNGSVORRICHTUNG, BEURTEILUNGSVERFAHREN UND
BEURTEILUNGSPROGRAMM

DISPOSITIF D'ÉVALUATION, PROCÉDÉ D'ÉVALUATION ET PROGRAMME D'ÉVALUATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.12.2018  JP 2018247350**

(43) Date of publication of application:
**29.09.2021  Bulletin 2021/39**

(73) Proprietor: **JVCKenwood Corporation
Yokohama-shi, Kanagawa 2210022 (JP)**

(72) Inventor: **SHUDO, Katsuyuki
Yokohama-shi, Kanagawa 221-0022 (JP)**

(74) Representative: **Schmidbauer, Andreas Konrad
Wagner & Geyer Partnerschaft mbB
Patent- und Rechtsanwälte
Gewürzmühlstrasse 5
80538 München (DE)**

(56) References cited:
WO-A1-2014/208761    WO-A1-2014/208761
WO-A1-2016/052646    WO-A1-2018/216347
WO-A1-2019/098173    JP-A- 2015 064 254
JP-A- 2017 158 866    JP-A- 2017 158 866
JP-A- 2018 140 007    US-A1- 2010 092 929

## Description

### Field

The present invention relates an evaluation device, an evaluation method, and an evaluation program.

### Background

In recent years, it is said that cognitive functional impairment and brain functional impairment are increasing, and a demand for detecting this type of cognitive functional impairment and brain functional impairment early and a demand for quantitatively evaluating severity of symptoms are required. It is known that symptoms of cognitive functional impairment and brain functional impairment affect cognitive ability. Therefore, an evaluation is performed on a subject based on cognitive ability of the subject. For example, an apparatus that displays multiple numbers, instructs a subject to add the numbers to obtain an answer, and checks the answer provided by the subject has been proposed (for example, see JP 2011-083403 A).

In the method described in JP 2011-083403 A or the like, the subject selects an answer by operating a touch panel or the like and it is thus difficult to obtain high evaluation accuracy due to a correct answer by chance or an error in operation performed by the subject.

JP 2017-158866A discloses a diagnosis support device capable of efficiently supporting diagnosis of a visual cognition disorder.

WO 2018/216347 A1 discloses an evaluation device, an evaluation method, and an evaluation program.

JP 2018-140007 A discloses accurate evaluation using a result of detection of a subject's visual line.

There have been demands for evaluating cognitive functional impairment and brain functional impairment with high accuracy.

### Summary

The invention is defined by the independent claims. The dependent claims define advantageous embodiments.

Accordingly, one aspect of the present invention has been conceived in light of the circumstances described above and an object thereof is to provide an evaluation device, an evaluation method, and an evaluation program capable of evaluating cognitive functional impairment and brain functional impairment with high accuracy.

According to one aspect, there is provided an evaluation device comprising: a display configured to display images; a gaze point detecting unit configured to detect a positional data of a gaze point of a subject who observes the display; a display controller configured to display, after presenting an instruction information to the subject, a specific target object that is an image and that is a correct answer to the instruction information and comparison target objects that are images and differ from the specific target object on the display; an area setting unit configured to set a specific area for the specific target object and comparison areas for the comparison target objects on the display; a determination unit configured to determine, based on the positional data of the gaze point, whether the gaze point is present in each of the specific area and the comparison areas; an arithmetic unit configured to calculate, based on a determination result by the determination unit, a gaze point transition data; and an evaluating unit configured to obtain, based on the gaze point transition data, an evaluation data of the subject.

According to one aspect, there is provided an evaluation method comprising: displaying images on a display; detecting a positional data of a gaze point of a subject who observes the display; displaying, after presenting an instruction information to the subject, a specific target object that is an image and that is a correct answer to the instruction information and comparison target objects that are images and differ from the specific target object on the display; setting, on the display, a specific area for the specific target object and comparison areas for the comparison target objects in the display; determining, based on the positional data of the gaze point, whether the gaze point is present in each of the specific area and the comparison areas; calculating, based on a determination result, a gaze point transition data; and obtaining, based on the gaze point transition data, an evaluation data of the subject.

According to one aspect, there is provided an evaluation program that causes a computer to execute a process comprising: a process of displaying images on a display; a process of detecting a positional data of a gaze point of a subject who observes the display; a process of displaying, after presenting an instruction information to the subject, a specific target object that is an image and that is a correct answer to the instruction information and comparison target objects that are images and differ from the specific target object on the display; a process of setting, on the display, a specific area for the specific target object and comparison areas for the comparison target objects in the display; a process of determining, based on the positional data of the gaze point, whether the gaze point is present in each of the specific area and the comparison areas; a process of calculating, based on a determination result, a gaze point transition data; and a process of obtaining, based on the gaze point transition data, an evaluation data of the subject.

According to an aspect of the present invention, it is possible to provide an evaluation device, an evaluation method, and an evaluation program capable of evaluating cognitive functional impairment and brain functional impairment with high accuracy.

**Brief Description of Drawings**

FIG. 1 is a perspective view schematically illustrating an example of a line-of-sight detecting device according to one embodiment;
FIG. 2 is a diagram illustrating an example of a hardware configuration of the line-of-sight detecting device according to the embodiment;
FIG. 3 is a functional block diagram illustrating an example of the line-of-sight detecting device according to the embodiment;
FIG. 4 is a schematic diagram illustrating a method for calculating a positional data of a corneal curvature center according to the embodiment;
FIG. 5 is a schematic diagram illustrating a method for calculating a positional data of the corneal curvature center according to the embodiment;
FIG. 6 is a schematic diagram illustrating an example of a calibration process according to the embodiment;
FIG. 7 is a schematic diagram illustrating an example of a gaze point detecting process according to the embodiment;
FIG. 8 is a diagram illustrating an example of a content that is displayed on a display in an instruction operation;
FIG. 9 is a diagram illustrating an example of a content that is displayed on the display in a target display operation;
FIG. 10 is a diagram illustrating an example of a guiding target object that guides a line of sight of a subject;
FIG. 11 is a diagram illustrating an example of the guiding target object that guides the line of sight of the subject;
FIG. 12 is a diagram illustrating another example of a content that is displayed on the display in the instruction operation;
FIG. 13 is a diagram illustrating another example of a content that is displayed on the display in the target display operation;
FIG. 14 is a diagram illustrating another example of a content that is displayed on the display in the instruction operation;
FIG. 15 is a diagram illustrating another example of a content that is displayed on the display in the target display operation;
FIG. 16 is a diagram illustrating another example of a content that is displayed on the display in the instruction operation;
FIG. 17 is a diagram illustrating another example of a content that is displayed on the display in the target display operation;
FIG. 18 is a flowchart illustrating an example of an evaluation method according to the embodiment; and
FIG. 19 is a flowchart illustrating an example of an evaluation process.

**Description of Embodiments**

Preferred embodiments of an evaluation device, an evaluation method, and an evaluation program according to the present disclosure will be described based on the drawings. Furthermore, the present invention is not limited to the embodiments. Furthermore, the components described in the embodiments include one that can easily be replaced by those skilled in the art or one that is substantially identical.

In a description below, the positional relationships among components will be described by setting a three-dimensional global coordinate system. It is assumed that a direction parallel to a first axis of a predetermined plane is defined as an X-axis direction, a direction parallel to a second axis of the predetermined plane orthogonal to the first axis is defined as a Y-axis direction, and a direction parallel to a third axis that is orthogonal to each of the first axis and the second axis is defined as a Z-axis direction. The predetermined plane includes an XY plane.

Line-of-sight detecting device

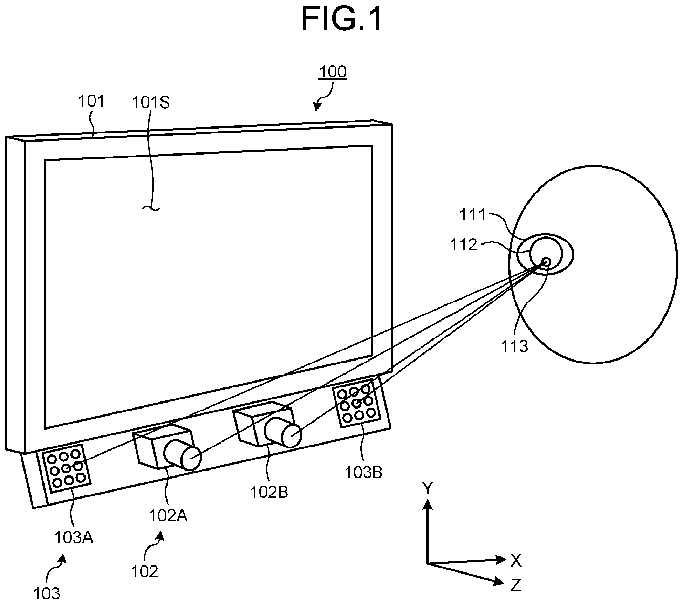

FIG. 1 is a perspective view schematically illustrating an example of a line-of-sight detecting device 100 according to one embodiment. The line-of-sight detecting device 100 is used as an evaluation device that evaluates cognitive functional impairment and brain functional impairment. As illustrated in FIG. 1, the line-of-sight detecting device 100 includes a display device 101, a stereo camera device 102, and an illuminating device 103.

The display device 101 includes a flat panel display, such as a liquid crystal display (LCD) or an organic electro-luminescence (EL) display (OLED). In the embodiment, the display device 101 includes a display 101S. The display 101S displays an image. In the embodiment, the display 101S displays an index for evaluating, for example, a visual performance of a subject. The display 101S is substantially parallel to the XY plane. The X-axis direction corresponds to the horizontal direction of the display 101S, the Y-axis direction corresponds to the vertical direction of the display 101S, and the Z-axis direction corresponds to the depth direction orthogonal to the display 101S.

The stereo camera device 102 includes a first camera 102A and a second camera 102B. The stereo camera device 102 is arranged below the display 101S of the display device 101. The first camera 102A and the second camera 102B are arranged in the X-axis direction. The first camera 102A is arranged in the negative X direction relative to the second camera 102B. Each of the first camera 102A and the second camera 102B includes an infrared camera and includes, an optical system capable of transmitting near-infrared light with a wavelength of, for example, 850 (nm) and an image sensor capable of receiving the near-infrared light.

The illuminating device 103 includes a first light source 103A and a second light source 103B. The illuminating device 103 is arranged below the display 101S of the display device 101. The first light source 103A and the second light source 103B are arranged in the X-axis direction. The first light source 103A is arranged in the negative direction relative to the first camera 102A. The second light source 103B is arranged in the positive direction relative to the second camera 102B. Each of the first light source 103A and the second light source 103B includes a light emitting diode (LED) light source and is able to emit near-infrared light with a wavelength of, for example, 850 (nm). Furthermore, the first light source 103A and the second light source 103B may also be arranged between the first camera 102A and the second camera 102B.

The illuminating device 103 emits near-infrared light that is detection light and illuminates an eyeball 111 of a subject. The stereo camera device 102 captures an image of a part of the eyeball 111 (hereinafter, referred to as an "eyeball" including the part of the eyeball) by the second camera 102B when the eyeball 111 is irradiated with the detection light emitted from the first light source 103A and captures an image of the eyeball 111 by the first camera 102A when the eyeball 111 is irradiated with the detection light emitted from the second light source 103B.

A frame synchronization signal is output from at least one of the first camera 102A and the second camera 102B. The first light source 103A and the second light source 103B output detection light based on the frame synchronization signal. The first camera 102A captures image data of the eyeball 111 when the eyeball 111 is irradiated with the detection light emitted from the second light source 103B. The second camera 102B captures image data of the eyeball 111 when the eyeball 111 is irradiated with the detection light emitted from the first light source 103A.

When the eyeball 111 is irradiated with the detection light, a part of the detection light is reflected at a pupil 112 and the light from the pupil 112 is incident into the stereo camera device 102. Furthermore, when the eyeball 111 is irradiated with the detection light, a corneal reflection image 113 that is a virtual image of a cornea is formed on the eyeball 111 and the light from the corneal reflection image 113 is incident into the stereo camera device 102.

By appropriately setting the relative position between a set of the first camera 102A and the second camera 102B and a set of the first light source 103A and the second light source 103B, the intensity of the light incident from the pupil 112 to the stereo camera device 102 is reduced and the intensity of the light incident from the corneal reflection image 113 to the stereo camera device 102 is increased. That is, the image of the pupil 112 captured by the stereo camera device 102 has a low luminance and the image of the corneal reflection image 113 has a high luminance. The stereo camera device 102 can detect the position of the pupil 112 and a position of the corneal reflection image 113 based on the luminance of the image captured.

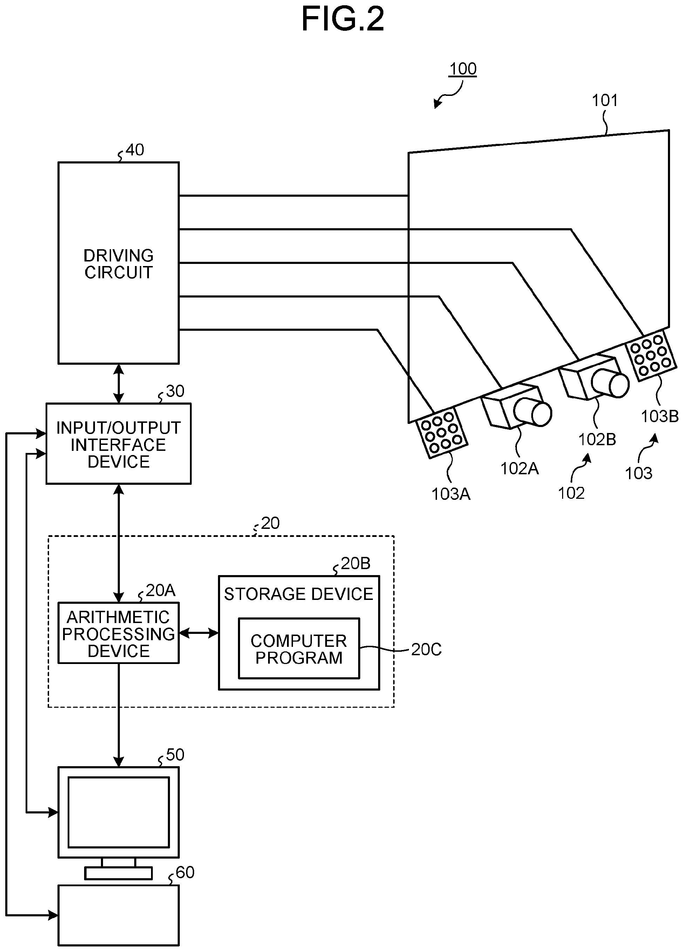

FIG. 2 is a diagram illustrating an example of a hardware configuration of the line-of-sight detecting device 100 according to the embodiment. As illustrated in FIG. 2, the line-of-sight detecting device 100 includes the display device 101, the stereo camera device 102, the illuminating device 103, a computer system 20, an input/output interface device 30, a driving circuit 40, an output device 50, and an input device 60.

The computer system 20, the driving circuit 40, the output device 50, and the input device 60 perform data communication via the input/output interface device 30. The computer system 20 includes an arithmetic processing device 20A and a storage device 20B. The arithmetic processing device 20A includes a microprocessor, such as a central processing unit (CPU). The storage device 20B includes a memory, such as a read only memory (ROM) and a random access memory (RAM), or storage. The arithmetic processing device 20A performs arithmetic processing in accordance with a computer program 20C that is stored in the storage device 20B.

The driving circuit 40 generates a driving signal and outputs the driving signal to the display device 101, the stereo camera device 102, and the illuminating device 103. Furthermore, the driving circuit 40 supplies the image data of the eyeball 111 captured by the stereo camera device 102 to the computer system 20 via the input/output interface device 30.

The output device 50 includes a display, such as a flat panel display. The output device 50 may also include a speaker that is able to output a voice. Furthermore, the output device 50 may also include a printer. The input device 60 generates input data by being operated. The input device 60 includes a keyboard or a mouse for a computer system. Furthermore, the input device 60 may also include a touch sensor arranged on the display of the output device 50 as a display.

In the embodiment, the display device 101 and the computer system 20 are separated devices. Furthermore, the display device 101 and the computer system 20 may also be integrated. For example, if the line-of-sight detecting device 100 includes a tablet type personal computer, the computer system 20, the input/output interface device 30, the driving circuit 40, and the display device 101 may also be mounted on the tablet type personal computer.

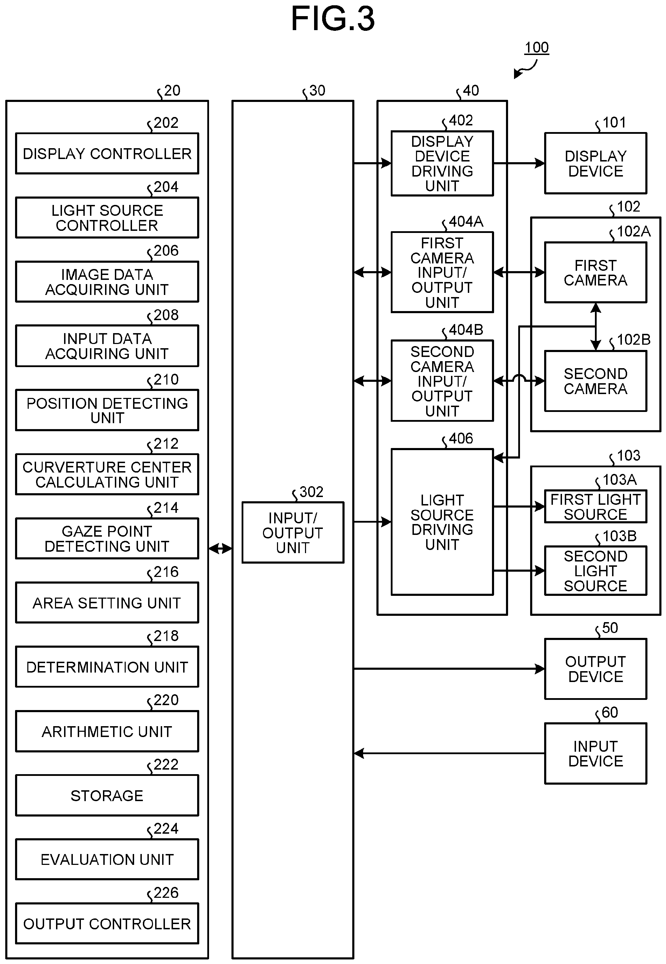

FIG. 3 is a functional block diagram illustrating an example of the line-of-sight detecting device 100 according to the embodiment. As illustrated in FIG. 3, the input/output interface device 30 includes an input/output unit 302. The driving circuit 40 includes a display device driving unit 402 that generates a driving signal for driving the display device 101 and that outputs the driving signal to the display device 101; a first camera input/output unit 404A that generates a driving signal for driving the first camera 102A and that outputs the driving signal to the first camera 102A; a second camera input/output unit 404B that generates a driving signal for driving the second camera 102B and that outputs the driving signal to the second camera 102B; and a light source driving unit 406 that generates a driving signal for driving the first light source 103A and the second light source 103B and that outputs the driving signal to the first light source 103A and the second light source 103B. Furthermore, the first camera input/output unit 404A supplies the image data of the eyeball 111 captured by the first camera 102A to the computer system 20 via the input/output unit 302. The second camera input/output unit 404B supplies the image data of the eyeball 111 captured by the second camera 102B to the computer system 20 via the input/output unit 302.

The computer system 20 controls the line-of-sight detecting device 100. The computer system 20 includes a display controller 202, a light source controller 204, an image data acquiring unit 206, an input data acquiring unit 208, a position detecting unit 210, a curvature center calculating unit 212, a gaze point detecting unit 214, an area setting unit 216, a determination unit 218, an arithmetic unit 220, a storage 222, an evaluation unit 224, and an output controller 226. The function of the computer system 20 is performed by the arithmetic processing device 20A and the storage device 20B.

The display control unit 202 is able to perform an instruction operation of displaying, on the display 101S, instruction information that is used to instruct the subject to gaze at an image of a pattern without displaying the image. In this case, the instruction information includes character information that is capable of allowing the subject to specify and imagine the pattern, such as a name of the pattern and a feature thereof. Moreover, the instruction information is not limited to the information that is displayed on the display 101S by the display control unit 202 but may also be, as will described later, information that is output by a voice by the output device 50, such as a speaker. The display control unit 202 performs a target display operation of displaying, after the instruction operation, a specific target object that is a pattern and that is a correct answer to the instruction described above, and comparison target objects each of which differs from the specific target object as multiple target objects on the display. Examples of the patterns corresponding to these target objects include a figure such as a polygon, an external appearance of a living thing such as an animal, or the like. Furthermore, characters may also be included in the pattern as the target object. Furthermore, a color, a design, or the like may also be included in the pattern as the target object.

For example, when it is assumed that the pattern that is to be gazed at by the subject is a predetermined polygonal pattern (for example, a pentagonal pattern), the display control unit 202 displays, in the target display operation, the predetermined polygonal pattern that is a correct answer as the specific target object, and displays polygonal patterns each of which has corners the number of which differs from that of the predetermined polygon as comparison target objects on the display. In this case, it is possible to set the comparison target objects such that the comparison target objects include a first comparison target object that is a polygonal pattern having corners the number of which differs from that of the specific target object by 1 and a second comparison target object that is a polygonal pattern having corners the number of which differs from that of the specific target object by greater than or equal to 2.

The light source controller 204 controls the light source driving unit 406 and controls an operation state of the first light source 103A and the second light source 103B. The light source controller 204 controls the first light source 103A and the second light source 103B such that the first light source 103A and the second light source 103B emit the detection light at different timings.

The image data acquiring unit 206 acquires, from the stereo camera device 102 via the input/output unit 302, the image data of the eyeball 111 of the subject captured by the stereo camera device 102 that includes the first camera 102A and the second camera 102B.

The input data acquiring unit 208 acquires, from the input device 60 via the input/output unit 302, the input data generated by an operation of the input device 60.

The position detecting unit 210 detects positional data of the pupil center based on the image data of the eyeball 111 acquired by the image data acquiring unit 206. Furthermore, the position detecting unit 210 detects positional data of the corneal reflection center based on the image data of the eyeball 111 acquired by the image data acquiring unit 206. The pupil center is a center of the pupil 112. The corneal reflection center is a center of the corneal reflection image 113. The position detecting unit 210 detects, for each of the left and right eyeballs 111 of the subject, the positional data of the pupil center and the positional data of the corneal reflection center.

The curvature center calculating unit 212 calculates positional data of a corneal curvature center of the eyeball 111 based on the image data of the eyeball 111 acquired by the image data acquiring unit 206.

The gaze point detecting unit 214 detects positional data of the gaze point P of the subject based on the image data of the eyeball 111 acquired by the image data acquiring unit 206. In the present embodiment, the positional data of the gaze point P indicates the positional data of an intersection point between a line-of-sight vector of the subject that is defined by the three-dimensional global coordinate system and the display 101S of the display device 101. The gaze point detecting unit 214 detects a line-of-sight vector of each of the right and left eyeballs 111 of the subject based on the positional data of the pupil center and the positional data of the corneal curvature center that are acquired from the image data of the eyeball 111. After the line-of-sight vector has been detected, the gaze point detecting unit 214 detects the positional data of the gaze point P indicating the intersection point between the line-of-sight vector and the display 101S.

The area setting unit 216 set, in the target display period for which the target display operation is being operated, a specific area for the specific target object and comparison areas for the comparison target objects on the display 101S. Moreover, when the comparison target objects include the first comparison target object and the second comparison target object, the area setting unit 216 is able to set, as the comparison area, a first comparison area for the first comparison target object and a second comparison area for the second comparison target object.

The determination unit 218 determines, in the target display period, based on the positional data of the gaze point P, whether the gaze point P is present in each of the task feature area and the instruction area, and then, outputs determination data. The determination unit 218 determines whether the gaze point P is present in each of the task feature area and the instruction area at, for example, regular intervals. The regular interval is, for example, a period (for example, every 20 (msec)) of a frame synchronization signal that is output from each of the first camera 102A and the second camera 102B.

The arithmetic unit 220 calculates, based on the determination data by the determination unit 218, a gaze point transition data that indicates a transition of the gaze point P in the target display period.

The gaze point transition data includes an arrival time data that indicates a time period from a start time of the target display period to an arrival time at which the gaze point arrives at the specific area, a movement frequency data that indicates the number of times of position movement of the gaze point P among the multiple comparison areas before the gaze point P first arrives at the specific area, a specific presence time data that indicates a presence time in which gaze point P is present in the specific area in the target display period, a comparison presence time data that indicates a presence time in which the gaze point P is present in the comparison areas in the display period, and a final area data that indicates an area in which the gaze point P is finally present among the specific area and the comparison areas in the display period. Moreover, when the first comparison area and the second comparison area are set, the comparison presence time data includes a first comparison presence time data that indicates a presence time in which the gaze point P is present in the first comparison area and a second comparison presence time data that indicates a presence time in which gaze point P is present in the second comparison areas.

Furthermore, the arithmetic unit 220 includes a management timer that manages a playback time of the video and a detection timer T1 that detects an elapsed time from displaying the video on the display 101S. The arithmetic unit 220 includes a counter that counts the number of determination that the gaze point P is present in the specific area and the comparison areas. When the first comparison area and the second comparison area are set, it may also be possible to have a configuration with counters each of which counts the number of determination that the gaze point P is present in the first comparison area and the second comparison area respectively.

The evaluating unit 224 is able to obtain an evaluation data of the subject based on the gaze point transition data. The evaluation data includes data that is used to evaluate, in the target display operation, whether the subject is able to gaze at the specific target object and the comparison target objects that are displayed on the display 101S.

The storage unit 222 stores therein the determination data, the gaze point transition data (the specific presence time data, the comparison presence time data, the movement frequency data, the final area data, and the arrival time data), and the evaluation data, which are described above. Furthermore, the storage unit 222 stores an evaluation program that causes a computer to execute a process of displaying an image on the display 101S; a process of detecting the positional data of the gaze point P of the subject who observes the display 101S; a process of displaying, after presenting an instruction content without displaying the image to be gazed at by the subject, the specific target object that is an image and that is a correct answer to the instruction and the comparison target objects each of which is different image from the specific target object on the display 101S; a process of setting, on the display 101S, the specific area for the specific target object and the comparison areas for the comparison target objects in the display 101S; a process of determining, based on the positional data of the gaze point P, whether the gaze point P is present in each of the specific area and the comparison areas; a process of calculating, based on a determination result, the gaze point transition data; and a process of obtaining, based on the gaze point transition data, the evaluation data of the subject.

The output control unit 226 outputs the data to at least one of the display device 101 and the output device 50. Furthermore, the output control unit 226 is able to perform the instruction operation of outputting, from the output device 50 such as a speaker, the instruction information that is used to instruct the subject to gaze at a pattern without displaying image information on the pattern.

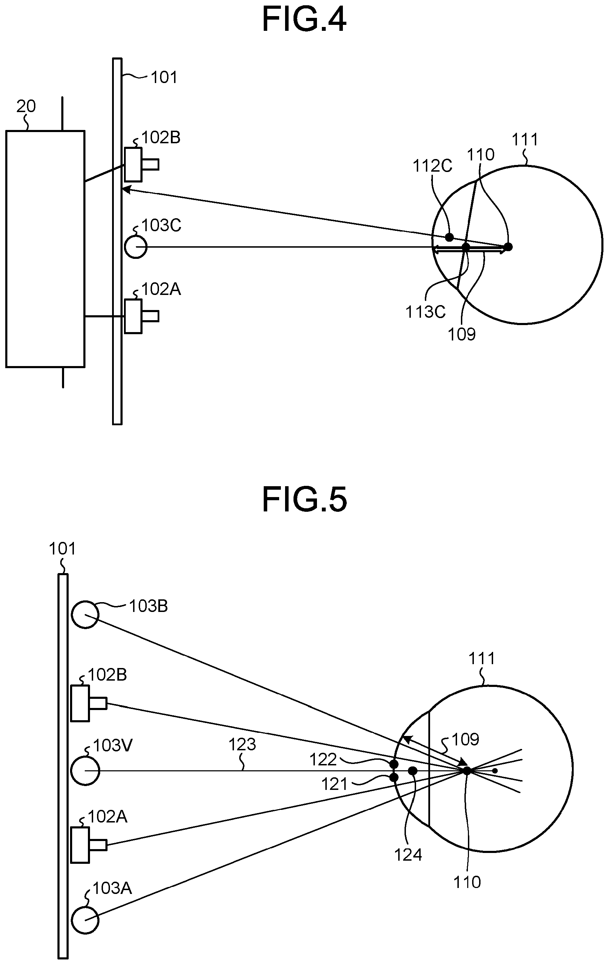

In the following, an outline of processes performed by the curvature center calculating unit 212 according to the embodiment will be described. The curvature center calculating unit 212 calculates the positional data of the corneal curvature center of the eyeball 111 based on the image data of the eyeball 111. Each of FIG. 4 and FIG. 5 is a schematic diagram illustrating a calculation method of positional data of a corneal curvature center 110 according to the embodiment. FIG. 4 illustrates an example in which the eyeball 111 is illuminated by a light source 103C. FIG. 5 illustrates an example in which the eyeball 111 is illuminated by the first light source 103A and the second light source 103B.

First, the example illustrated in FIG. 4 will be described. The light source 103C is arranged between the first camera 102A and the second camera 102B. A pupil center 112C is a center of the pupil 112. A corneal reflection center 113C is a center of the corneal reflection image 113. In FIG. 4, the pupil center 112C indicates a pupil center when the eyeball 111 is illuminated by the single light source 103C. The corneal reflection center 113C indicates a corneal reflection center when the eyeball 111 is illuminated by the single light source 103C. The corneal reflection center 113C is present on a straight line connecting the light source 103C and a corneal curvature center 110. The corneal reflection center 113C is positioned at a middle point between a cornea surface and the corneal curvature center 110. A corneal curvature radius 109 is a distance between the cornea surface and the corneal curvature center 110. Positional data of the corneal reflection center 113C is detected by the stereo camera device 102. The corneal curvature center 110 is present on a straight line connecting the light source 103C and the corneal reflection center 113C. The curvature center calculating unit 212 calculates, as the positional data of the corneal curvature center 110, positional data of a position which is located at a predetermined distance from the corneal reflection center 113C on the straight line. The predetermined value is a value that is determined in advance from a curvature radius value of a general cornea or the like and is stored in the storage 222.

In the following, the example illustrated in FIG. 5 will be described. In the embodiment, a set of the first camera 102A and the second light source 103B and a set of the second camera 102B and the first light source 103A are arranged at bilaterally symmetrical positions with respect to a straight line that passes through an intermediate position between the first camera 102A and the second camera 102B. It is assumed that a virtual light source 103V is present at the intermediate position between the first camera 102A and the second camera 102B. A corneal reflection center 121 indicates a corneal reflection center in an image that is obtained by capturing the eyeball 111 by the second camera 102B. A corneal reflection center 122 indicates a corneal reflection center in an image that is obtained by capturing the eyeball 111 by the first camera 102A. A corneal reflection center 124 indicates a corneal reflection center associated with the virtual light source 103V. Positional data of the corneal reflection center 124 is calculated based on positional data of the corneal reflection center 121 and positional data of the corneal reflection center 122 that are captured by the stereo camera device 102. The stereo camera device 102 detects the positional data of the corneal reflection center 121 and the positional data of the corneal reflection center 122 in the three-dimensional local coordinate system that is defined in the stereo camera device 102. A camera calibration using a stereo calibration method is performed in advance on the stereo camera device 102, and a transformation parameter for transforming the three dimensional local coordinate system of the stereo camera device 102 into the three-dimensional global coordinate system is calculated. The transformation parameter is stored in the storage 222. The curvature center calculating unit 212 transforms the positional data of the corneal reflection center 121 and the positional data of the corneal reflection center 122 captured by the stereo camera device 102 into the positional data in the three-dimensional global coordinate system by using the transformation parameter. The curvature center calculating unit 212 calculates the positional data of the corneal reflection center 124 in the three-dimensional global coordinate system based on the positional data of the corneal reflection center 121 and the positional data of the corneal reflection center 122 that are defined in the three-dimensional global coordinate system. The corneal curvature center 110 is present on a straight line 123 connecting the virtual light source 103V and the corneal reflection center 124. The curvature center calculating unit 212 calculates, as the positional data of the corneal curvature center 110, positional data of a position which is located at a predetermined distance from the corneal reflection center 124 on the straight line 123. The predetermined value is a value that is determined in advance from a curvature radius value of a general cornea or the like and is stored in the storage 222.

In this way, even when two light sources are present, the corneal curvature center 110 is calculated by the same method as the method that is used when a single light source is present.

The corneal curvature radius 109 corresponds to a distance between the cornea surface and the corneal curvature center 110. Accordingly, the corneal curvature radius 109 is calculated by calculating the positional data of the cornea surface and the positional data of the corneal curvature center 110.

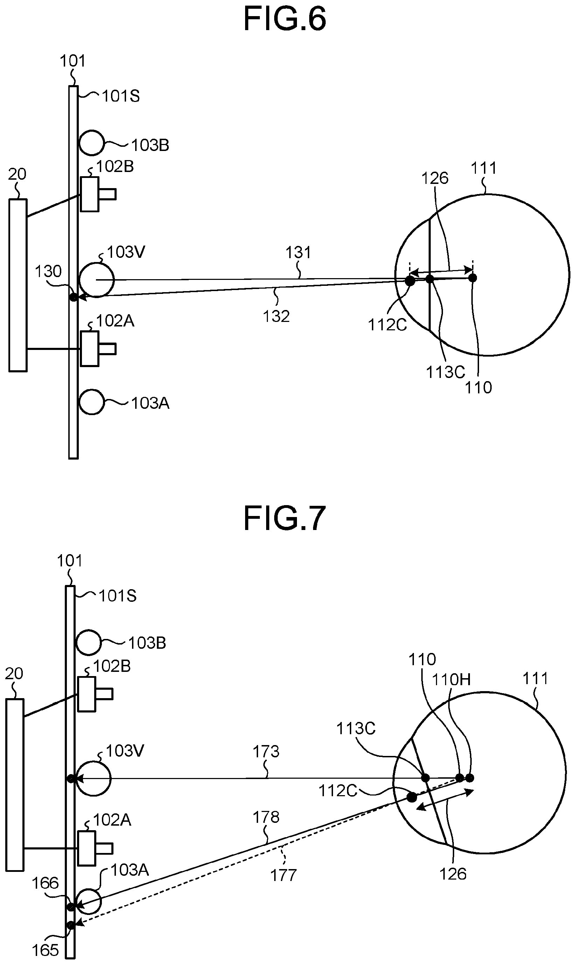

In the following, an example of a line-of-sight detecting method according to the embodiment will be described. FIG. 6 is a schematic diagram illustrating an example of a calibration process according to the embodiment. In the calibration process, a target position 130 is set in order to allow the subject to gaze steadily. The target position 130 is defined in the three-dimensional global coordinate system. In the embodiment, the target position 130 is set at, for example, a middle position of the display 101S of the display device 101. Furthermore, the target position 130 may also be set at an edge position of the display 101S. The output controller 226 displays a target image at the set target position 130. A straight line 131 is a straight line connecting the virtual light source 103V and the corneal reflection center 113C. A straight line 132 is a straight line connecting the target position 130 and the pupil center 112C. The corneal curvature center 110 is an intersection point between the straight line 131 and the straight line 132. The curvature center calculating unit 212 can calculate the positional data of the corneal curvature center 110 based on the positional data of the virtual light source 103V, the positional data of the target position 130, the positional data of the pupil center 112C, and the positional data of the corneal reflection center 113C.

In the following, a gaze point detecting process performed by the gaze point detecting unit 214 will be described. The gaze point detecting process is performed after the calibration process. The gaze point detecting unit 214 calculates a line-of-sight vector of the subject and positional data of the gaze point P based on the image data of the eyeball 111. FIG. 7 is a schematic diagram illustrating an example of the gaze point detecting process according to the embodiment. In FIG. 7, a gaze point 165 indicates a gaze point P that is obtained from the corneal curvature center calculated using a general curvature radius value. A gaze point 166 indicates a gaze point P that is obtained from the corneal curvature center calculated using a distance 126 obtained in the calibration process. The pupil center 112C indicates the pupil center calculated in the calibration process, and the corneal reflection center 113C indicates the corneal reflection center calculated in the calibration process. A straight line 173 is a straight line connecting the virtual light source 103V and the corneal reflection center 113C. The corneal curvature center 110 corresponds to a position of the corneal curvature center that is calculated from a general curvature radius value. The distance 126 is a distance between the pupil center 112C and the corneal curvature center 110 calculated in the calibration process. A corneal curvature center 110H indicates a corrected position of the corneal curvature center that has been corrected by using the distance 126. The corneal curvature center 110H is obtained under a condition that the corneal curvature center 110 is present on the straight line 173 and the distance between the pupil center 112C and the corneal curvature center 110 is the distance 126. Accordingly, a line of sight 177 that is calculated in a case of using the general curvature radius value is corrected to a line of sight 178. Furthermore, the gaze point P on the display 101S of the display device 101 is corrected from the gaze point 165 to the gaze point 166.

Evaluation method

In the following, the evaluation method according to the embodiment will be described. In the evaluation method according to the embodiment, a developmental disability is evaluated as a visual performance of the subject by using the line-of-sight detecting device 100 described above.

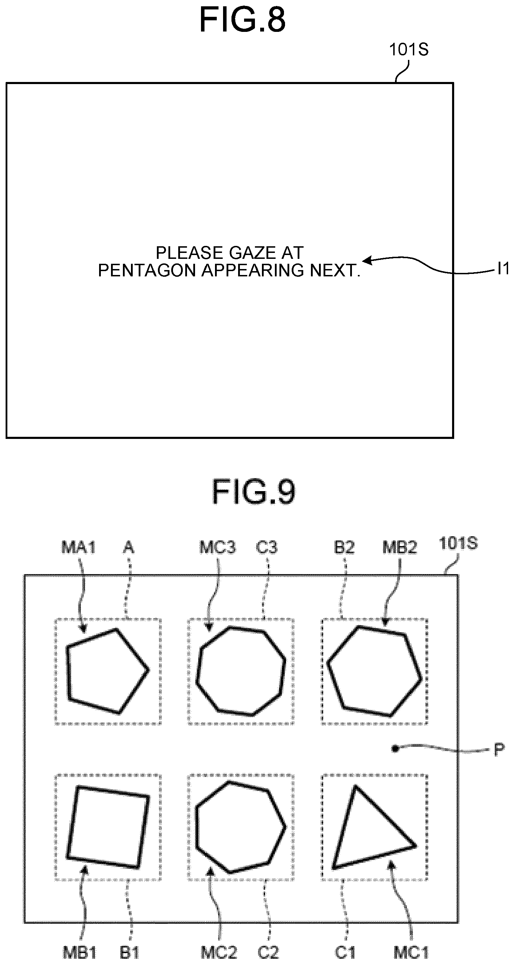

FIG. 8 is a diagram illustrating an example of a content that is displayed on the display 101S in the instruction operation. As illustrated in FIG. 8, the display control unit 202 displays, in the instruction operation, for example, instruction information I1 that is used to give an instruction to the subject on the display 101S for a predetermined period. For a period of time for which the instruction information I1 is displayed on the display 101S, the subject is instructed to imagine a pattern. In this case, the instruction information I1 indicates, as an example, a task with a content that instructs to select a pattern that indicates a "pentagon". In the embodiment, in order to instruct the subject to imagine a "pentagon", the instruction information I1 is constituted by only the character information. Namely, in the instruction information I1, no image information on a pattern that indicates a "pentagon" is included. Furthermore, in the embodiment, in the instruction operation, a case in which the instruction information I1 is displayed on the display 101S is used as an example. However, the case is not limited thereto. In the instruction operation, for example, the output control unit 226 may also output, in addition to the display of the instruction information I1, or instead of the display of the instruction information I1, a voice corresponding to the instruction information I1 from the speaker.

FIG. 9 is a diagram illustrating an example of a content that is displayed on the display 101S in the target display operation. As illustrated in FIG. 9, the display control unit 202 displays, on the display 101S, the specific target object that is a correct answer to the instruction information I1 and multiple comparison target objects that are incorrect answers to the instruction information I1. In the example illustrated in FIG. 9, the display control unit 202 displays, as a specific target object MA1, the pentagonal pattern that is a correct answer to the instruction information I1. Furthermore, the display control unit 202 displays, as the comparison target objects, each of the patterns of a triangle, a quadrilateral, a hexagon, a heptagon, and an octagon that are incorrect answers to the instruction information I1. Here, a quadrilateral pattern and a hexagonal pattern each having corners, the number of which differs from the number of corners (5 corners) of the pentagon that is a correct answer by 1, are represented as the comparison target objects MB1 and MB2, respectively. Furthermore, a triangular pattern, a heptagonal pattern, and an octagonal pattern each having corners, the number of which differs from that of the pentagon by greater than or equal to 2, are represented as the comparison target objects MC1, MC2, and MC3, respectively.

Furthermore, in the target display period by the display control unit 202, the area setting unit 216 sets a specific area A for the specific target object MA1 that is a correct answer to the instruction information I1. The area setting unit 216 is able to set the specific area A in an area that includes at least a part of the specific target object MA1. In the embodiment, the area setting unit 216 sets the specific area A in a rectangular area that includes the specific target object MA1.

Furthermore, the area setting unit 216 sets the comparison areas B1, B2, and C1 to C3 for the comparison target objects MB1, MB2, and MC1 to MC3 that are incorrect answers to the instruction information I1. The area setting unit 216 separately sets, for the comparison areas B1, B2, and C1 to C3, the first comparison areas B1 and B2 for the first comparison target objects MB1 and MB2, respectively, and the second comparison areas C1 to C3 for the second comparison target objects MC1 to MC3, respectively.

The area setting unit 216 is able to set each of the first comparison areas B1 and B2 in an area that includes at least a part of the first comparison target objects MB1 and MB2, respectively. Furthermore, the area setting unit 216 is able to set each of the second comparison areas C1 to C3 in an area that includes at least a part of the second comparison target objects MC1 to MC3, respectively. In the embodiment, the area setting unit 216 sets the first comparison areas B1 and B2 and the second comparison areas C1 to C3 in the rectangular areas that include the first comparison target objects MB1 and MB2 and the second comparison target objects MC1 to MC3, respectively. Furthermore, the area setting unit 216 sets, on the display 101S, the specific area A, the first comparison areas B1 and B2, and the second comparison areas C1 to C3 so as not to overlap with each other.

Moreover, the specific area A, the first comparison areas B1 and B2, and the second comparison areas C1 to C3 are not displayed on the display 101S. The shape of each of the specific area A, the first comparison areas B1 and B2, and the second comparison areas C1 to C3 is not limited to a rectangle described above and the shape thereof may also be another shape such as a circular shape, an elliptical shape, or a polygonal shape.

Furthermore, FIG. 9 illustrates an example of the gaze point P that is displayed on the display 101S as a result of, for example, measurement; however, in practice, the gaze point P is not displayed on the display 101S. Detection of the positional data of the gaze point P is performed in, for example, a period (for example, every 20 (msec)) of a frame synchronization signal that is output from the first camera 102A and the second camera 102B. The first camera 102A and the second camera 102B capture an image in synchronization.

It is known that symptoms of cognitive functional impairment and brain functional impairment affect cognitive ability and memory ability of the subject. If the subject does not have cognitive functional impairment and brain functional impairment, in the instruction operation, the subject is able to imagine a pentagonal pattern by viewing the instruction information I1, view the multiple target objects MA1, MB1, MB2, and MC1 to MC3 that are displayed on the display 101S in the target display operation, and distinctively find out the specific target object MA1 that is the pentagonal pattern.

In contrast, if the subject has cognitive functional impairment and brain functional impairment, in the instruction operation, in some cases, the subject is not able to imagine the pentagonal pattern by viewing the instruction information I1 and is not able to gaze at the specific target object MA1. Therefore, in the embodiment, the pattern itself that is to be gazed at by the subject is not displayed by the instruction information I1, and alternatively, by presenting an instruction to the subject by using character information, voice information, or the like for the pattern in a predetermined period, the subject is instructed to imagine the pattern. After presenting the instruction information I1 in the predetermined period, by displaying the multiple target objects MA1, MB1, MB2, and MC1 to MC3 including the specific target object MA1 that is a correct answer to the instruction, the subject is instructed to perform selection thereof. In this temporal flow, a period of time for which the subject views the instruction information I1 to imagine the pattern is ensured and, after that, the subject is instructed to select the imagined answer. Then, based on the gaze point transition data while the subject finds out the answer, a possibility of cognitive functional impairment and brain functional impairment of the subject is evaluated.

Moreover, as in the embodiment, with a method for displaying the multiple target objects MA1, MB1, MB2, and MC1 to MC3 on the display 101S, there may be a case in which, at the start of the target display operation, the gaze point P of the subject is accidentally placed at the specific target object MA1 or the like that is a correct answer. In such a case, since there is a possibility of determining that the subject found the correct answer regardless of whether the subject is cognitive functional impairment and brain functional impairment, it is difficult to evaluate the subject with high accuracy. Accordingly, for example, it is possible to evaluate the subject by performing the following processes.

First, as the target display operation, the multiple target objects (the specific target object MA1 and the comparison target objects MB1, MB2, and MC1 to MC3) are displayed. In this case, it is possible to evaluate the subject from a viewpoint whether the subject gazes at the multiple target objects one by one, whether the subject is able to finally reach the specific target object MA1 that is a correct answer, how long does it take before the subject reaches the specific target object MA1, and whether the subject is able to gaze at the specific target object MA1.

In the target display operation, when the positional data of the gaze point P of the subject is detected, the determination unit 218 determines whether the gaze point P of the subject is present in the specific area A and the comparison areas B1, B2, and C1 to C3, and then, outputs a determination data.

The arithmetic unit 220 calculates, based on the determination data, the gaze point transition data that indicates the transition of the gaze point P in the display period. The arithmetic unit 220 calculates, as the gaze point transition data, the presence time data, the movement frequency data, the final area data, and the arrival time data.

The presence time data includes the specific presence time data that indicates the presence time in which the gaze point P is present in the specific area A, the first comparison presence time data that indicates the presence time in the gaze point P is present in the first comparison areas B1 and B2, and the second comparison presence time data that indicates the presence time in which the gaze point P is present in the second comparison areas C1 to C3. In the embodiment, after having determined by the determination unit 218 whether the gaze point P is present in the specific area A, the first comparison areas B1 and B2, and the second comparison areas C1 to C3 at, for example, regular intervals, it is possible to estimate that the presence time in which the gaze point P is present in each of the specific area A, the first comparison areas B1 and B2, and the second comparison areas C1 to C3 is longer as the number of determination that the gaze point P is present is increased. Therefore, the presence time data can be assumed to be the number of the determination by the determination unit 218 that the gaze point P is present in the specific area A, the first comparison areas B1 and B2, and the second comparison areas C1 to C3. In other words, the arithmetic unit 220 uses a count value CNTA of the counter as the specific presence time data, uses a count value CNTB as the first comparison presence time data, and uses a count value CNTC as the second comparison presence time data.

Furthermore, the movement frequency data indicates the number of times of position movement of the gaze point P among the multiple comparison areas B1, B2, and C1 to C3 before the gaze point P first arrives at the specific area A. Therefore, the arithmetic unit 220 is able to count the number of times of position movement of the gaze point P between the areas of the specific area A and the comparison areas B1, B2, and C1 to C3, and use the count result before the gaze point P arrives at the specific area A as the movement frequency data.

Furthermore, the final area data indicates an area in which the gaze point P is finally present among the specific area A and the comparison areas B1, B2, and C1 to C3 in the display time, i.e., an area that is finally gazed at by the subject as the answer. The arithmetic unit 220 updates the area in which the gaze point P is present every time the gaze point P is detected and is thus able to use the detection result at an end of the display period as the final area data.

Furthermore, the arrival time data indicates a period of time from the start time of the display period to an arrival time at which the gaze point P first arrives at the specific area A. Therefore, by measuring an elapsed time from the start of the display period by the timer T1 and detecting a measurement value of the timer T1 when the gaze point P first arrives at the specific area A with setting a flag value to 1, the arithmetic unit 220 is able to use a detection result of the timer T1 as the arrival time data.

In the embodiment, the evaluating unit 224 obtains the evaluation data based on the presence time data, the movement frequency data, the final area data, and the arrival time data.

Here, a data value based on the final area data is denoted by D1, a data value based on the specific presence time data is denoted by D2, a data value based on the first comparison presence time data is denoted by D3, a data value based on the second comparison presence time data is denoted by D4, a data value based on the arrival time data is denoted by D5, and a data value based on the movement frequency data is denoted by D6. However, it is assumed that the data value D1 based on the final area data is set to 1 if the gaze point P of the subject is finally present in the specific area A (i.e., in a case of a correct answer), and set to 0 if the gaze point P of the subject is finally not present in the specific area A (i.e., in a case of an incorrect answer). Moreover, in a case in which the gaze point P of the subject is finally not present in the specific area A (in a case of an incorrect answer), the data value D1 based on the final area data may also be set to 0.5 if the gaze point P is finally present in the first comparison areas B1 and B2 and may also be set to 0 if the gaze point P is finally present in the second comparison areas C1 to C3. Furthermore, it is assumed that the data value D2 based on the specific presence time data is the number of seconds in which the gaze point P is present in the specific area A. Furthermore, it is assumed that the data value D3 based on the first comparison presence time data is the number of seconds in which the gaze point P is present in the first comparison areas B1 and B2. Furthermore, it is assumed that the data value D4 based on the second comparison presence time data is the number of seconds in which the gaze point P is present in the second comparison areas C1 to C3. Moreover, regarding the data values D2, D3, and D4, it may also be possible to set an upper limit value that is a smaller number of seconds than the display period. Furthermore, the data value D5 based on the arrival time data is set to an inverse number of the arrival time (for example, 1/(arrival time)/10) (10 is a coefficient for setting an arrival time evaluation value to 1 or less based on an assumption that a minimum value of the arrival time is 0.1 second). Furthermore, the counter value is used as it is as the data value D6 based on the movement frequency data. Moreover, it may also be possible to appropriately set an upper limit value for the data value D6.

In this case, an evaluation value ANS is represented by, for example, as follows;

$$ANS = D1 \cdot K1 + D2 \cdot K2 - D3 \cdot K3 - D4 \cdot K4 + D5 \cdot K5 + D6 \cdot K6$$

K1 to K6 are constants for weighting. The constant K1 to K6 may be appropriately set.

A value of the evaluation value ANS represented by Expression above becomes large when the data value D1 based on the final area data is large, when the data value D2 based on the specific presence time data is large, when the data value D3 based on the first comparison presence time data is small, when the data value D4 based on the second comparison presence time data is small, when the data value D5 based on the arrival time data is large, and when the data value D6 based on the movement frequency data is large. In other words, the evaluation value ANS becomes larger when the final gaze point P is present in the specific area A, when the presence time of the gaze point P in the specific area A is longer, when the presence time of the gaze point P in the first comparison areas B1 and B2 and the second comparison areas C1 to C3 is shorter, when the arrival time at which the gaze point P arrives at the specific area A after the start time of the display period is shorter, and when the number of times of position movement of the gaze point P between the areas is greater.

In contrast, the value of the evaluation value ANS becomes smaller when the data value D1 based on the final area data is small, when the data value D2 based on the specific presence time data is small, when the data value D3 based on the first comparison presence time data is large, when the data value D4 based on the second comparison presence time data is large, when the data value D5 based on the arrival time data is small, and when the data value D6 based on the movement frequency data is small. In other words, the evaluation value ANS becomes smaller when the final gaze point P is not present in the specific area A, when the presence time of the gaze point P in the specific area A is shorter, when the presence time of the gaze point P in the first comparison areas B1 and B2 and the second comparison areas C1 to C3 is longer, when the arrival time at which the gaze point P arrives at the specific area A after the start time of the display period is longer, and when the number of times of position movement of the gaze point P between the areas is smaller.

Therefore, the evaluating unit 224 is able to obtain the evaluation data by determining whether the evaluation value ANS is greater than or equal to a predetermined value. For example, when the evaluation value ANS is greater than or equal to the predetermined value, it is possible to evaluate that the subject is less likely to have cognitive functional impairment and brain functional impairment. Furthermore, when the evaluation value ANS is less than the predetermined value, it is possible to evaluate that the subject is highly likely to have cognitive functional impairment and brain functional impairment.

Furthermore, the evaluating unit 224 is able to store the evaluation value ANS in the storage unit 222. For example, it may also be possible to cumulatively store the evaluation value ANS for the same subject and perform evaluation by comparing with the past evaluation values. For example, when the evaluation value ANS is higher than the past evaluation value, it is possible to evaluate that a cognitive function has improved as compared with the evaluation obtained last time. Furthermore, when a cumulative value of the evaluation value ANS is gradually increased, it is possible to evaluate that the cognitive function has gradually improved.

Furthermore, the evaluating unit 224 may also be able to perform evaluation by using the presence time data, the movement frequency data, the final area data, and the arrival time data individually or in combination. For example, when the gaze point P accidentally arrives at the specific area A while the multiple target objects are viewed, the data value D6 based on the movement frequency data becomes small. In this case, it is possible to perform evaluation together with the data value D2 based on the specific presence time data described above. For example, when the movement frequency is small but the presence time in the specific area A is long, it is possible to evaluate that the subject is able to gaze at the specific area A that is a correct answer. Furthermore, when the movement frequency is small and the presence time is also short, it is possible to evaluate that the gaze point P has accidentally passed through the specific area A.

Furthermore, when the movement frequency is small and the final area is the specific area A, it is possible to evaluate that, for example, the gaze point arrives at the specific area A that is the correct answer with a smaller movement frequency. In contrast, when the movement frequency described above is small and the final area is not the specific area A, it is possible to evaluate that, for example, the gaze point P has accidentally passed through the specific area A.

Furthermore, the comparison target objects MB1 and MB2 for the first comparison areas B1 and B2, respectively, are the patterns each of which indicates a figure having corners the number of which differs from that of the specific target object MA1 that is a correct answer by 1. There is a possibility that the subject erroneously gazes at these patterns even if the subject does not have cognitive functional impairment and brain functional impairment. In contrast, the comparison target objects MC1 to MC3 for the second comparison areas C1 to C3, respectively, are the patterns each of which indicates a figure having corners the number of which differs from that of the specific target object MA1 that is a correct answer by greater than or equal to 2. It can be said that, when the subject does not have cognitive functional impairment and brain functional impairment, the subject is less likely to erroneously gaze at these patterns. Accordingly, it may also be possible to apply weightings such that a coefficient K3 of the first comparison presence time data is smaller than the coefficient K4 of the second comparison presence time data (K3<K4).

In the embodiment, when the evaluation unit 224 outputs the evaluation data, the output controller 226 is able to allow the output device 50 to output, in accordance with the evaluation data, character data indicating that, for example, "it seems that the subject is less likely to have cognitive functional impairment and brain functional impairment" or character data indicating that "it seems that the subject is highly likely to have cognitive functional impairment and brain functional impairment". Furthermore, when the evaluation value ANS for the same subject becomes higher than the past evaluation value ANS, the output controller 226 is able to allow the output device 50 to output character data indicating that "a cognitive function and a brain function have improved" or the like.

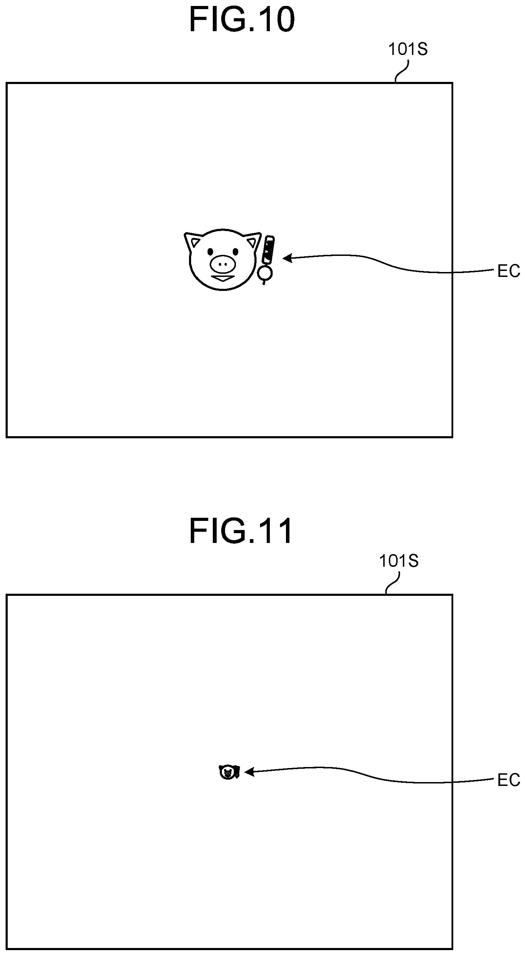

FIG. 10 and FIG. 11 are diagrams each of which illustrates an example of a guiding target object EC that guides a line of sight of the subject. The display control unit 202 is able to display, on the display 101S, the guiding target object EC between, for example, the instruction operation and the target display operation. In this case, first, as illustrated in FIG. 10, the display control unit 202 displays the guiding target object EC in a predetermined size at a center of the display 01S. Then, as illustrated in FIG. 11, the display control unit 202 displays, on the display 101S, the guiding target object EC so as to become gradually smaller toward a place in which the line of sight of the subject is desired to be guided (for example, the center of the display 101S). By displaying the guiding target object EC in this way, it is possible to guide the line of sight of the subject to the desired position on the display 101S.

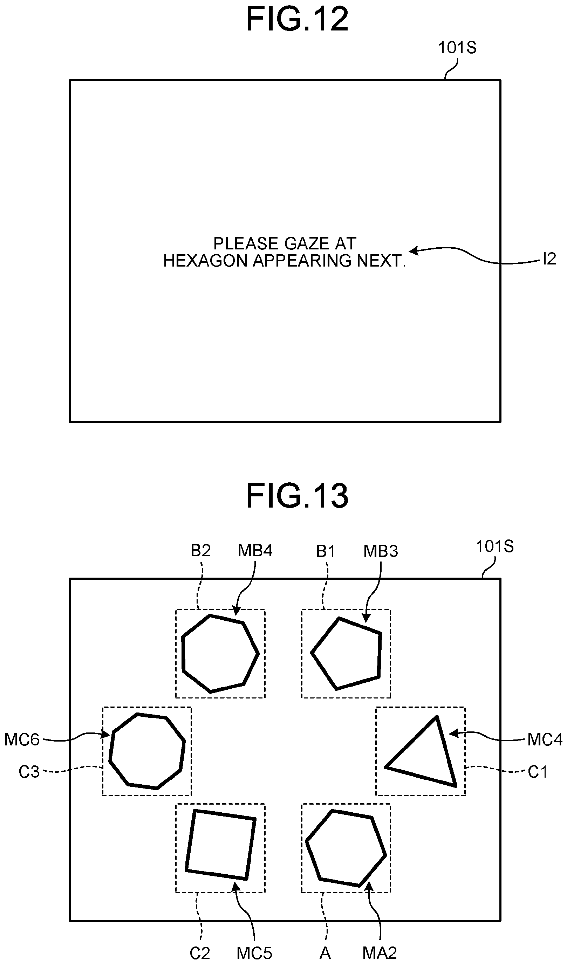

FIG. 12 is a diagram illustrating another example of a content that is displayed on the display 101S in the instruction operation. In the example illustrated in FIG. 12, a task with a content indicating that the display control unit 202 instructs the user to select the pattern that indicates a "hexagon" as the instruction information I2 is illustrated. In the embodiment, in order to instruct the subject to imagine a "hexagon", the instruction information I2 is constituted by only the character information. Namely, an image information on the pattern that indicates a "hexagon" is not included in the instruction information I2. Moreover, similarly to the above description, in addition to the display on the display 101S or instead of the display on the display 101S, a voice of the instruction information I2 may also be output from a speaker.

FIG. 13 is a diagram illustrating another example of a content that is displayed on the display 101S in the target display operation. In the example illustrated in FIG. 13, the display control unit 202 displays, as a specific target object MA2, a hexagonal pattern that is a correct answer to the instruction information I2. Furthermore, the display control unit 202 displays, as comparison target objects, each of the patterns of a triangle, a quadrilateral, a pentagon, a heptagon, and an octagon that are incorrect answers to the instruction information I2. Here, a pentagonal pattern and a heptagonal pattern each of which has corners, the number of which differs from the number of corners (6 corners) of the hexagon that is a correct answer by 1, are denoted by the comparison target objects MB3 and MB4, respectively. Furthermore, a triangle pattern, a quadrilateral pattern, and an octagonal pattern each of which has corners, the number of which differs from the number of the hexagon by greater than or equal to 2, are denoted by the comparison target objects MC4, MC5, and MC6, respectively. Moreover, in the example illustrated in FIG. 13, the display control unit 202 is able to arrange each of the target objects at a position that does not overlap with the position in which the line of sight has been guided by using the guiding target object EC as illustrated in FIG. 10 and FIG. 11. In this case, as illustrated in FIG. 13 the display control unit 202 arranges each of the target objects on the circumference around the center of the display 101S. However, the arrangement is not limited thereto and another arrangement may also be used.

Furthermore, in the target display period based on the display control unit 202, the area setting unit 216 sets the specific area A for the specific target object MA2 that is a correct answer to the instruction information I2. The area setting unit 216 is able to set the specific area A in an area that includes at least a part of the specific target object MA2. In the embodiment, the area setting unit 216 sets the specific area A in a rectangular area that includes the specific target object MA2.

Furthermore, the area setting unit 216 sets the comparison areas B1, B2, and C1 to C3 for the comparison target objects MB3, MB4, and MC4 to MC6, respectively, that are incorrect answers to the instruction information I2. The area setting unit 216 separately sets, for the comparison areas B1, B2, and C1 to C3, the first comparison areas B1 and B2 for the first comparison target objects MB3 and MB4, respectively, and the second comparison areas C1 to C3 for the second comparison target objects MC4 to MC6, respectively, as the comparison areas.

The area setting unit 216 is able to set each of the first comparison areas B1 and B2 in an area that includes at least a part of the first comparison target objects MB3 and MB4, respectively. Furthermore, the area setting unit 216 is able to set each of the second comparison areas C1 to C3 in an area that includes at least a part of the second comparison target objects MC4 to MC6, respectively. In the embodiment, the area setting unit 216 sets each of the first comparison areas B1 and B2 and the second comparison areas C1 to C3 in the rectangular area that includes the first comparison target objects MB3 and MB4 and the second comparison target objects MC4 to MC6, respectively. Furthermore, the area setting unit 216 sets, on the display 101S, the specific area A, the first comparison areas B1 and B2, and the second comparison areas C1 to C3 so as not to overlap with each other.

Moreover, the specific area A, the first comparison areas B1 and B2, and the second comparison areas C1 to C3 are not displayed on the display 101S. The shape of the specific area A, the first comparison areas B1 and B2, and the second comparison areas C1 to C3 is not limited to a rectangle described above and the shape thereof may also be another shape, such as a circular shape, an elliptical shape, or a polygonal shape.

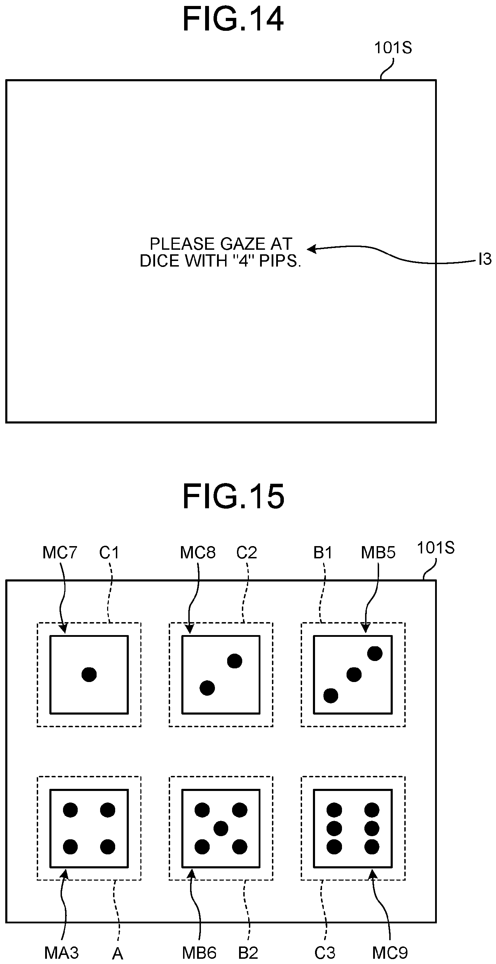

FIG. 14 is a diagram illustrating another example of a content that is displayed on the display 101S in the instruction operation. In the example illustrated in FIG. 14, a task with a content indicating that the display control unit 202 instructs the user to select the pattern of a dice with "4" spots as the instruction information I3 is illustrated. In the embodiment, in order to instruct the subject to imagine a pattern corresponding to the dice with "4" spots, the instruction information I3 is constituted by only the character information. Namely, an image information on the pattern that indicates the dice with "4" spots is not included in the instruction information I3. Moreover, similarly to the above description, in addition to the display on the display 101S or instead of the display on the display 101S, a voice of the instruction information I3 may also be output from a speaker.

FIG. 15 is a diagram illustrating another example of a content that is displayed on the display 101S in the target display operation. In each of the examples described above, the descriptions have been given of a case, as an example, in which the subject is instructed to imagine a polygon. However, the case is not limited thereto. In the example illustrated in FIG. 15, the display control unit 202 may also display, as a specific target object MA3, the pattern of the dice with "4" spots that is a correct answer to the instruction information I3. Furthermore, the display control unit 202 displays, as the comparison target objects, each of the patterns of the dice with 1 spot, 2 spots, 3 spots, 5 spots, and 6 spots that are incorrect answers to the instruction information I3. Here, the patterns of the dice with 3 spots and 5 spots which differ from the number of spots 4 of the dice with 4 spots which is a correct answer by 1 are denoted by comparison target objects MB5 and MB6, respectively. Furthermore, the patterns of the dice with 1 spot, 2 spots, and 6 spots which differ from the number of spots 4 of the dice with 4 spots which is a correct answer by greater than or equal to 2 are denoted by comparison target objects MC7, MC8, and MC9, respectively.

Furthermore, in the target display period based on the display control unit 202, the area setting unit 216 sets the specific area A for the specific target object MA3 that is a correct answer to the instruction information I3. The area setting unit 216 is able to set the specific area A in an area that includes at least a part of the specific target object MA3. In the embodiment, the area setting unit 216 sets the specific area A in a rectangular area that includes the specific target object MA3.

Furthermore, the area setting unit 216 sets, for the comparison areas B1, B2, and C1 to C3, the comparison areas B1, B2, and C1 to C3 for the comparison target objects MB5, MB6, and MC7 to MC9, respectively, that are incorrect answers to the instruction information I3. The area setting unit 216 separately sets, as the comparison areas, the first comparison areas B1 and B2 for the first comparison target objects MB5 and MB6, respectively, and the second comparison areas C1 to C3 for the second comparison target objects MC7 to MC9, respectively.

The area setting unit 216 is able to set each of the first comparison areas B1 and B2 in an area that includes at least a part of the first comparison target objects MB5 and MB6, respectively. Furthermore, the area setting unit 216 is able to set each of the second comparison areas C1 to C3 in an area that includes at least a part of the second comparison target objects MC7 to MC9, respectively. In the embodiment, the area setting unit 216 sets each of the first comparison areas B1 and B2 and the second comparison areas C1 to C3 in the rectangular area that includes the first comparison target objects MB5 and MB6 and the second comparison target objects MC7 to MC9, respectively. Furthermore, the area setting unit 216 sets, on the display 101S, the specific area A, the first comparison areas B1 and B2, the second comparison areas C1 to C3 so as not to overlap with each other.

Moreover, the specific area A, the first comparison areas B1 and B2, and the second comparison areas C1 to C3 are not displayed on the display 101S. The shape of the specific area A, the first comparison areas B1 and B2, and the second comparison areas C1 to C3 is not limited to a rectangle described above and the shape thereof may also be another shape, such as a circular shape, an elliptical shape, or a polygonal shape.

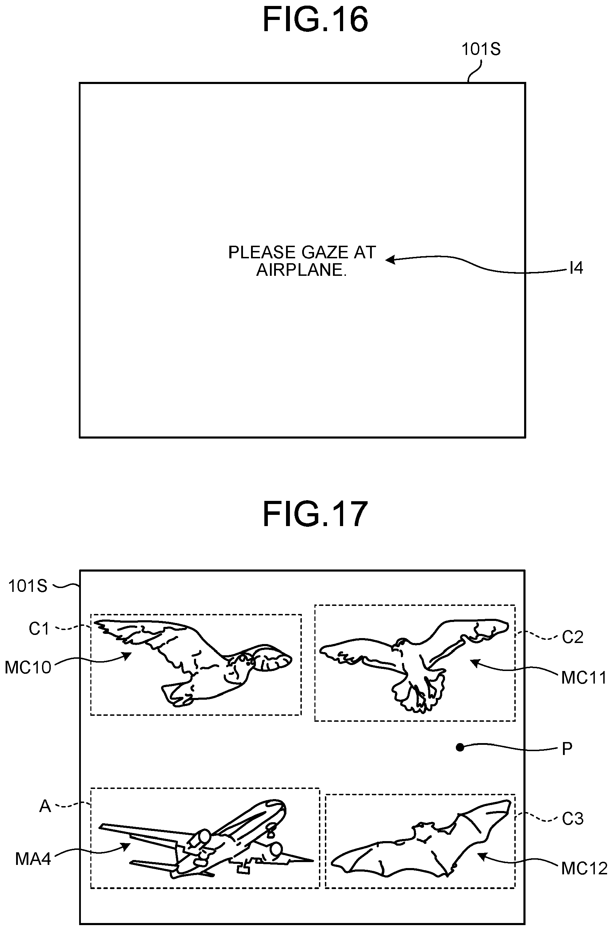

FIG. 16 is a diagram illustrating another example of a content that is displayed on the display 101S in the instruction operation. In each of the examples described above, the descriptions have been given of a case, as an example, in which the subject is instructed to imagine a pattern that includes a numerical concept. However the case is not limited thereto. In the example illustrated in FIG. 16, the display control unit 202 may also display, as the instruction information I4, a task with a content that instructs the user to select a pattern that is an "airplane". In the embodiment, in order to instruct the subject to imagine a pattern corresponding to an "airplane", the instruction information I4 is constituted by only the character information. Namely, the pattern that corresponds to an "airplane" is not included in the instruction information I4. Moreover, similarly to the above description, in addition to the display on the display 101S or instead of the display on the display 101S, a voice of the instruction information I4 may also be output from a speaker.

FIG. 17 is a diagram illustrating an example of another content that is displayed on the display 101S in the target display operation. In the example illustrated in FIG. 17, the display control unit 202 displays, as a specific target object MA4, a pattern of the airplane that is a correct answer to the instruction information I4. Furthermore, the display control unit 202 displays, as second comparison target objects MC10 to MC12, patterns of two kinds of birds and a pattern of a bat that are incorrect answers to the instruction information I4. In this way, in the example illustrated in FIG. 17, it is possible not to set the first comparison target object. However, an example is not limited thereto and different types of comparison target objects may also be used.

Furthermore, in the target display period based on the display control unit 202, the area setting unit 216 sets the specific area A for the specific target object MA4 that is a correct answer to the instruction information I4. The area setting unit 216 is able to set the specific area A in the area that includes at least a part of the specific target object MA4. In the embodiment, the area setting unit 216 the sets the specific area A in a rectangular area that includes the specific target object MA4.

Furthermore, the area setting unit 216 sets the comparison areas C1 to C3 for the comparison target objects MC10 to MC12, respectively, that are incorrect answers to the instruction information I4. The area setting unit 216 is able to set each of the comparison areas C1 to C3 in an area that includes at least a part of the comparison target objects MC10 to MC12, respectively. In the embodiment, the area setting unit 216 sets each of the comparison areas C1 to C3 in a rectangular area that includes the comparison target objects MC10 to MC12, respectively. Furthermore, the area setting unit 216 sets, on the display 101S, the specific area A and the comparison areas C1 to C3 so as not to overlap with each other.

Moreover, the specific area A and the comparison areas C1 to C3 are not displayed on the display 101S. The shape of each of the specific area A and the comparison areas C1 to C3 is not limited to a rectangle described above and the shape thereof may also be another shape, such as a circular shape, an elliptical shape, or a polygonal shape.

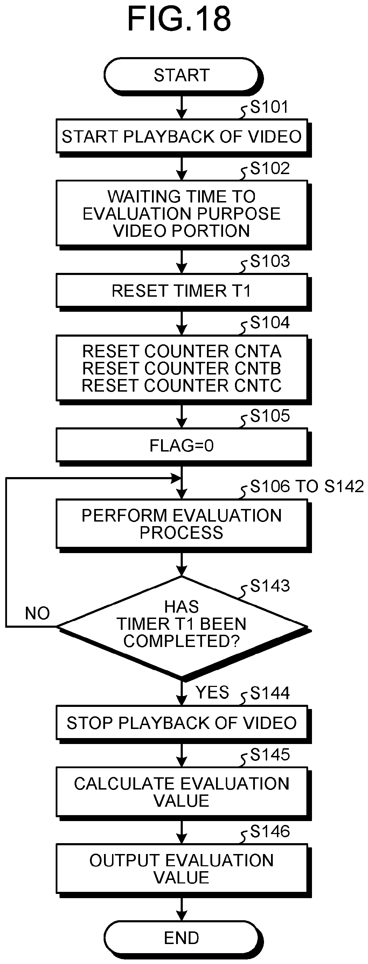

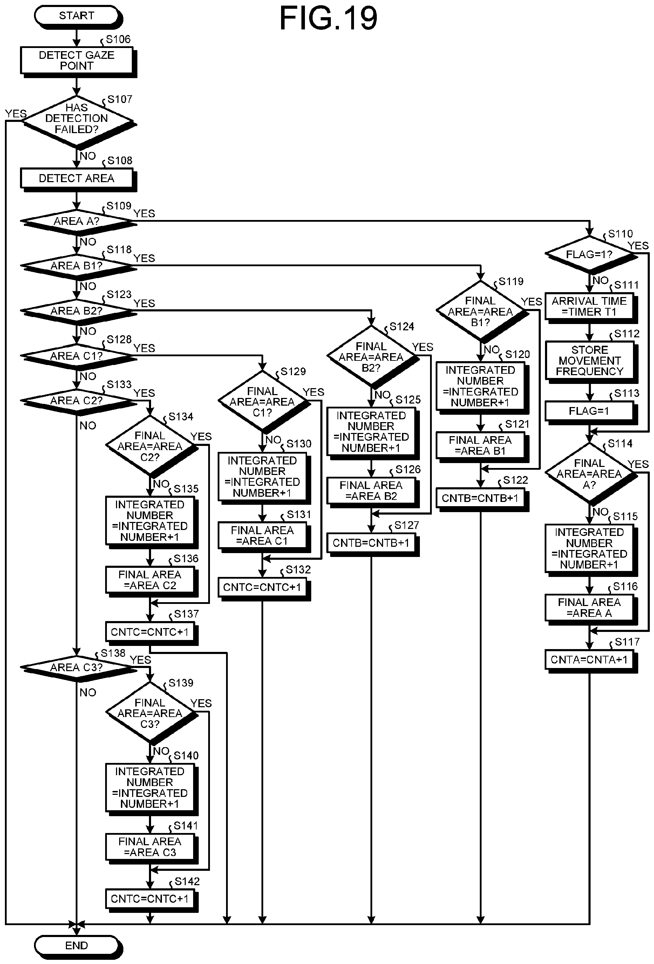

In the following, an example of the evaluation method according to the embodiment will be described with reference to FIG. 18 and FIG. 19. FIG. 18 is a flowchart illustrating an example of the evaluation method according to the embodiments. In the evaluation method according to the embodiments, as illustrated in FIG. 18, first, the display control unit 202 starts a playback of the video (Step S101). After an elapse of a waiting time to an evaluation purpose video portion (Step S102), the display control unit 202 resets the timer T1 (Step S103), resets count values CNTA, CNTB, and CNTC of the counters (Step S104), sets a flag value to 0 to clear the movement frequency data and the final area (Step S105).

After Step S105, an evaluation processes (Step S106 to Step S142) are performed. FIG. 19 is a flowchart illustrating an example of the evaluation process. As illustrated in FIG. 19, in the evaluation process, the gaze point detecting unit 214 detects a positional data of the gaze point P of the subject on the display 101S of the display device 101 for a predetermined sampling period (for example, 20 (msec)) while showing the video displayed on the display device 101 to the subject (Step S106). When the positional data has been detected (No at Step S107), the determination unit 218 determines, based on the positional data, an area in which the gaze point P is present (Step S108). Furthermore, when the positional data is not detected (Yes at Step S107), the process at Step S143 (see FIG. 18) and the subsequent processes, which will be described later, are performed.

When it is determined that the gaze point P is present in the specific area A (Yes at Step S109), the arithmetic unit 220 determines whether the flag value is 1, i.e., whether the gaze point P first arrives at the specific area A (1: has already arrived, 0: has not arrived yet) (Step S110). When the flag value is 1 (Yes at Step S110), the arithmetic unit 220 skips the subsequent processes at Step S111 to Step S113 and performs the process at Step S114 that will be described later.

Furthermore, when the flag value is not 1, i.e., when the gaze point P arrives at the specific area A for the first time (No at Step S110), the arithmetic unit 220 extracts a measurement result of the timer T1 as an arrival time data (Step S111). Furthermore, the arithmetic unit 220 allows the storage 222 to store a movement frequency data that indicates the movement frequency of the gaze point P between the areas before the gaze point P arrives at the specific area A (Step S112). After that, the arithmetic unit 220 changes the flag value to 1 (Step S113) .

Then, the arithmetic unit 220 determines whether the area in which the gaze point P is present at the latest detection, i.e., the final area, is the specific area A (Step S114). When the arithmetic unit 220 determines that the final area is the specific area A (Yes at Step S114), the arithmetic unit 220 skips the subsequent processes at Step S115 and Step S116 and performs the process at Step S117 that will be described later. Furthermore, when it is determined that the final area is not the specific area A (No at Step S114), the arithmetic unit 220 increments an integrated number that indicates the movement frequency of the gaze point P among the areas by 1 (Step S115), and changes the final area to the specific area A (Step S116). Furthermore, the arithmetic unit 220 increments the count value CNTA that indicates the presence time data in the specific area A by 1 (Step S117). After that, the arithmetic unit 220 performs the process at Step S143 (see FIG. 18) and the subsequent processes that will be described later.

Furthermore, when it is determine that the gaze point P is not present in the specific area A (No at Step S109), the arithmetic unit 220 determines whether the gaze point P is present in the first comparison area B1 (Step S118). When it is determined that the gaze point P is present in the first comparison area B1 (Yes at Step S118), the arithmetic unit 220 determines whether the area in which the gaze point P is present at the latest detection, i.e., the final area, is the first comparison area B1 (Step S119). When the arithmetic unit 220 determines that the final area is the first comparison area B1 (Yes at Step S119), the arithmetic unit 220 skips the subsequent processes at Step S120 and Step S121 and performs the process at Step S122 that will be described later. Furthermore, when it is determined that the final area is not the first comparison area B1 (No at Step S119), the arithmetic unit 220 increments an integrated number that indicates the movement frequency of the gaze point P among the areas by 1 (Step S120), and changes the final area to the first comparison area B1 (Step S121). Furthermore, the arithmetic unit 220 increments the count value CNTB that indicates the presence time data in the first comparison area B1 by 1 (Step S122). After that, the arithmetic unit 220 performs the process at Step S143 (see FIG. 18) and the subsequent processes that will be described later.

Furthermore, when it is determined that the gaze point P is not present in the first comparison area B1 (No at Step S118), the arithmetic unit 220 determines whether the gaze point P is present in the first comparison area B2 (Step S123). When it is determined that the gaze point P is present in the first comparison area B2 (Yes at Step S123), the arithmetic unit 220 determines whether the area in which the gaze point P is present at the latest detection, i.e., the final area, is the first comparison area B2 (Step S124). When the arithmetic unit 220 determines that the final area is present in the first comparison area B2 (Yes at Step S124), the arithmetic unit 220 skips the subsequent processes at Step S125 and Step S126 and performs the process at Step S127 that will be described later. Furthermore, when it is determined that the final area is not the first comparison area B2 (No at Step S124), the arithmetic unit 220 increments an integrated number that indicates the movement frequency of the gaze point P among the areas by 1 (Step S125), and changes the final area to the first comparison area B2 (Step S126). Furthermore, the arithmetic unit 220 increments the count value CNTB that indicates the presence time data in the first comparison area B2 by 1 (Step S127). After that, the arithmetic unit 220 performs the process at Step S143 (see FIG. 18) and the subsequent processes that will be described later.

Furthermore, when it is determined that the gaze point P is not present in the first comparison area B2 (No at Step S123), the arithmetic unit 220 determines whether the gaze point P is present in the second comparison area C1 (Step S128). When it is determined that the gaze point P is present in the second comparison area C1 (Yes at Step S128), the arithmetic unit 220 determines whether the area in which the gaze point P is present at the latest detection, i.e., the final area, is the second comparison area C1 (Step S129). When the arithmetic unit 220 determines that the final area is the second comparison area C1 (Yes at Step S129), the arithmetic unit 220 skips the subsequent processes at Step S130 and Step S131 and performs the process at Step S132 that will be described later. Furthermore, when it is determined that the final area is not the second comparison area C1 (No at Step S129), the arithmetic unit 220 increments an integrated number that indicates the movement frequency of the gaze point P among the areas by 1 (Step S130), and changes the final area to the second comparison area C1 (Step S131). Furthermore, the arithmetic unit 220 increments the count value CNTC that indicates the presence time data in the second comparison area C1 by 1 (Step S132). After that, the arithmetic unit 220 performs the process at Step S143 (see FIG. 18) and the subsequent process that will be described later.

Furthermore, when it is determined that the gaze point P is not present in the second comparison area C1 (No at Step S128), the arithmetic unit 220 determines whether the gaze point P is present in the second comparison area C2 (Step S133). When it is determine that the gaze point P is present in the second comparison area C2 (Yes at Step S133), the arithmetic unit 220 determines whether the area in which the gaze point P is present at the latest detection, i.e., the final area, is the second comparison area C2 (Step S134). When the arithmetic unit 220 determines that the final area is the second comparison area C2 (Yes at Step S134), the arithmetic unit 220 skips the subsequent processes at Step S135 and Step S136 and performs the process at Step S137 that will be described later. Furthermore, when it is determined that the final area is not the second comparison area C2 (No at Step S134), the arithmetic unit 220 increments an integrated number that indicates the movement frequency of the gaze point P among the areas by 1 (Step S135), and changes the final area to the second comparison area C2 (Step S136). Furthermore, the arithmetic unit 220 increments the count value CNTC that indicates the presence time data in the second comparison area C2 by 1 (Step S137). After that, the arithmetic unit 220 performs the process at Step S143 (see FIG. 18) and the subsequent processes which will be described later.

Furthermore, when it is determined that the gaze point P is not present in the second comparison area C2 (No at Step S133), the arithmetic unit 220 determines whether the gaze point P is present in the second comparison area C3 (Step S138). When it is determined that the gaze point P is present in the second comparison area C3 (Yes at Step S138), the arithmetic unit 220 determines whether the area in which the gaze point P is present at the latest detection, i.e., the final area, is the second comparison area C3 (Step S139). When the arithmetic unit 220 determines that the final area is the second comparison area C3 (Yes at Step S139), the arithmetic unit 220 skips the subsequent processes at Step S140 and Step S141 and performs the process at Step S142 that will be described later. Furthermore, when it is determined that the final area is not the second comparison area C3 (No at Step S139), the arithmetic unit 220 increments an integrated number that indicates the movement frequency of the gaze point P among the areas by 1 (Step S140), and changes the final area to the second comparison area C3 (Step S141). Furthermore, the arithmetic unit 220 increments the count value CNTC that indicates the presence time data in the second comparison area C3 by 1 (Step S142). After that, the arithmetic unit 220 performs the process at Step S143 (see FIG. 18) and the subsequent processes that will be described later.

After that, as illustrated in FIG. 18, the arithmetic unit 220 determines, based on the detection result of the detection timer T1, whether the time reaches a completion time of the playback of the video (Step S143). When it is determined, by the arithmetic unit 220, that the time does not reach the completion time of the playback of the video (No at Step S143), the arithmetic unit 220 repeatedly performs the process at Step S106 and the subsequent processes described above.

After Step S143, when it is determined, by the arithmetic unit 220, that the time reaches the completion time of the playback of the video (Yes at Step S143), the display control unit 202 stops the playback of the video (Step S144). After the playback of the video is stopped, the evaluating unit 224 calculates an evaluation value ANS based on the presence time data, the movement frequency data, the final area data, and the arrival time data that are obtained from the processing result described above (Step S145), and obtains an evaluation data based on the evaluation value ANS. After that, the output control unit 226 outputs the evaluation data obtained by the evaluating unit 224 (Step S146).

As described above, the evaluation device 100 according to the embodiments includes a display 101S configured to display images; a gaze point detecting unit 214 configured to detect a positional data of a gaze point P of a subject who observes the display 101S; a display controller 202 configured to display, after presenting an instruction information to the subject, a specific target object that is an image and that is a correct answer to the instruction information and comparison target objects that are images and differ from the specific target object on the display 101S; an area setting unit 216 configured to set a specific area A for the specific target object and comparison areas B1, B2, C1 to C3 for the comparison target objects on the display 101S; a determination unit 218 configured to determine, based on the positional data of the gaze point P, whether the gaze point P is present in each of the specific area A and the comparison areas B1, B2, C1 to C3; an arithmetic unit 220 configured to calculate, based on a determination result, a gaze point transition data; and an evaluating unit 224 configured to obtain, based on the gaze point transition data, an evaluation data of the subject.

Furthermore, the evaluation method according to the embodiment includes displaying images on a display 101S; detecting a positional data of a gaze point P of a subject who observes the display 101S; displaying, after presenting an instruction information to the subject, a specific target object that is an image and that is a correct answer to the instruction information and comparison target objects that are images and differ from the specific target object on the display 101S; setting, on the display 101S, a specific area A for the specific target object and comparison areas B1, B2, C1 to C3 for the comparison target objects in the display 101S; determining, based on the positional data of the gaze point P, whether the gaze point P is present in each of the specific area A and the comparison areas B1, B2, C1 to C3; calculating, based on a determination result, a gaze point transition data; and obtaining, based on the gaze point transition data, an evaluation data of the subject.

Furthermore, the evaluation program according to the embodiment causes a computer to execute a process comprising: a process of displaying images on a display 101S; a process of detecting a positional data of a gaze point of a subject who observes the display 101S; a process of displaying, after presenting an instruction information to the subject, a specific target object that is an image and that is a correct answer to the instruction information and comparison target objects that are images and differ from the specific target object on the display 101S; a process of setting, on the display 101S, a specific area A for the specific target object and comparison areas B1, B2, C1 to C3 for the comparison target objects in the display 101S; a process of determining, based on the positional data of the gaze point P, whether the gaze point P is present in each of the specific area A and the comparison areas B1, B2, C1 to C3; a process of calculating, based on a determination result, a gaze point transition data; and a process of obtaining, based on the gaze point transition data, an evaluation data of the subject.

According to the embodiments, by instructing the subject to gaze at the pattern without displaying image information on the pattern, it is possible to instruct the subject to imagine the pattern. Consequently, since a possibility of cognitive functional impairment and brain functional impairment of the subject is evaluated based on the gaze point transition at the time at which the subject finds out the imagined answer, it is possible to evaluate the subject with high accuracy. Furthermore, since it is possible to obtain the evaluation data of the subject based on the gaze point transition in the display period, it is possible to evaluate the subject with higher accuracy. Therefore, the evaluation device 100 is able to evaluate the subject with high accuracy.

Furthermore, in the evaluation device 100 according to the embodiments, the pattern to be gazed at by the subject is a polygonal pattern, and the display control unit 202 displays, on the display 101S, the specific target object that is the polygonal pattern and that is a correct answer to the instruction and the comparison target object that is a polygonal pattern having corners, the number of which differs from the number of the specific target object. Consequently, by instructing the subject to gaze at the target object that is easily imagined by the subject as a task, it is possible to evaluate a possibility of cognitive functional impairment and brain functional impairment of the subject with high accuracy.

Furthermore, in the evaluation device 100 according to the embodiments, the comparison target objects include the first comparison target object that is a polygonal pattern having corners, the number of which differs from the number of the specific target object by 1, and the second comparison target object that is a polygonal pattern having corners, the number of which differs from the number of the specific target object by greater than or equal to 2. The area setting unit 216 sets, as the comparison areas, the first comparison areas B1 and B2 for the first comparison target objects and the second comparison areas C1 to C3 for the second comparison target objects. The gaze point transition data includes the first comparison presence time data that indicates the presence time in which the gaze point P is present in the first comparison areas B1 and B2 and the second comparison presence time data that indicates the presence time in which the gaze point is present in the second comparison areas C1 to C3. And the evaluating unit 224 obtains the evaluation data by applying different weightings between the first comparison presence time data and the second comparison presence time data. Therefore, it is possible to evaluate a possibility of cognitive functional impairment brain functional impairment of the subject in stages with high accuracy. Furthermore, with this configuration, it is possible to make a difference in the evaluation for the subject who does not have cognitive functional impairment brain functional impairment between a case in which the subject gazes at the target object that is likely to be erroneously gazed at and a case in which the subject gazes at the target object that is less likely to be erroneously gazed at. Therefore, it is possible to evaluate a possibility of cognitive functional impairment and brain functional impairment of the subject with high accuracy.

The technical scope of the present embodiments is not limited to the embodiments described above and various modifications are possible. For example, in each of the embodiments, a case has been described as one example in which the evaluation device 100 is used as an evaluation device that evaluates a possibility of cognitive functional impairment and brain functional impairment. However, the embodiment is not limited thereto. For example, the evaluation device 100 may also be used as an evaluation device that evaluates a subject who does not have cognitive functional impairment and brain functional impairment.

**Industrial Applicability**

It is possible to use the evaluation device, the evaluation method, and the evaluation program according to the present embodiment in, for example, a line-of-sight detecting device.

**Claims**

1. An evaluation device (100) comprising:

   a display (101S) configured to display images;
   a gaze point detecting unit (214) configured to detect a positional data of a gaze point (P) of a subject who observes the display (101S);
   a display controller (202) configured to display, after presenting an instruction information (I1 to I4) to the subject, a specific target object (MA1 to MA4) that is an image and that is a correct answer to the instruction information (I1 to I4) and comparison target objects (MB1 to MB6, MC1 to MC12) that are images and differ from the specific target object (MA1 to MA4) on the display (101S);
   an area setting unit (216) configured to set a specific area (A) for the specific target object (MA1 to MA4) and comparison areas (B1 to B2, C1 to C3) for the comparison target objects (MB1 to MB6, MC1 to MC12) on the display (101S);
   a determination unit (218) configured to determine, based on the positional data of the gaze point (P), whether the gaze point (P) is present in each of the specific area (A) and the comparison areas (B1 to B2, C1 to C3);
   an arithmetic unit (220) configured to calculate, based on a determination result by the determination unit (218), a gaze point transition data; and
   an evaluating unit (224) configured to obtain, based on the gaze point transition data, an evaluation data of the subject; wherein
   the image that is to be gazed at by the subject is a polygonal pattern;
   the display control unit (202) is further configured to display, on the display (101S), the specific target object (MA1, MA2) that is the polygonal pattern and that is the correct answer to the instruction information (I1 to I4) and the comparison target objects (MB1 to MB4, MC1 to MC6) each of which is the polygonal pattern having corners, a number of which differs from that of the specific target object (MA1, MA2);
   the comparison target objects include at least one first comparison target object (MB1 to MB4) that is a polygonal pattern having corners, the number of which differs from that of the specific target object (MA1, MA2) by 1, and at least one second comparison target object (MC1 to MC6) that is a polygonal pattern having corners, the number of which differs from that of the specific target object (MA1, MA2) by greater than or equal to 2;
   the area setting unit (216) is further configured to set, as the comparison areas, at least one first comparison area (B1 to B2) for the at least one first comparison target object (MB1 to MB4) and at least one second comparison area (C1 to C3) for the at least one second comparison target object (MC1 to MC6);
   the gaze point transition data further includes a first comparison presence time data that indicates a presence time in which the gaze point (P) is present in the at least one first comparison area (B1 to B2) and a second comparison presence time data that indicates a presence time in which the gaze point (P) is present in the at least one second comparison area (C1 to C3); and
   the evaluating unit (224) is further configured to obtain the evaluation data by applying weightings (K1 to K6) between the first comparison presence time data and the second comparison presence time data.

2. An evaluation method comprising:

   displaying images on a display (101S);

detecting a positional data of a gaze point (P) of a subject who observes the display (101S);

displaying, after presenting an instruction information (I1 to I4) to the subject, a specific target object (MA1 to MA4) that is an image and that is a correct answer to the instruction information (I1 to I4) and comparison target objects (MB1 to MB6, MC1 to MC12) that are images and differ from the specific target object (MA1 to MA4) on the display (101S);

setting, on the display (101S), a specific area (A) for the specific target object (MA1 to MA4) and comparison areas (B1 to B2, C1 to C3) for the comparison target objects (MB1 to MB6, MC1 to MC12) in the display (101S);

determining, based on the positional data of the gaze point (P), whether the gaze point (P) is present in each of the specific area (A) and the comparison areas (B1 to B2, C1 to C3);

calculating, based on a determination result, a gaze point transition data; and

obtaining, based on the gaze point transition data, an evaluation data of the subject wherein

the image that is to be gazed at by the subject is a polygonal pattern;

at the displaying, displaying, on the display (101S), the specific target object (MA1, MA2) that is the polygonal pattern and that is the correct answer to the instruction information (I1 to I4) and the comparison target objects (MB1 to MB4, MC1 to MC6) each of which is the polygonal pattern having corners, a number of which differs from that of the specific target object (MA1, MA2);

the comparison target objects include at least one first comparison target object (MB1 to MB4) that is a polygonal pattern having corners, the number of which differs from that of the specific target object (MA1, MA2) by 1, and at least one second comparison target object (MC1 to MC6) that is a polygonal pattern having corners, the number of which differs from that of the specific target object (MA1, MA2) by greater than or equal to 2;

at the setting, setting, as the comparison areas, at least one first comparison area (B1 to B2) for the at least one first comparison target object (MB1 to MB4) and at least one second comparison area (C1 to C3) for the at least one second comparison target object (MC1 to MC6);

the gaze point transition data further includes a first comparison presence time data that indicates a presence time in which the gaze point (P) is present in the at least one first comparison area (B1 to B2) and a second comparison presence time data that indicates a presence time in which the gaze point (P) is present in the at least one second comparison area (C1 to C3); and

at the obtaining, obtaining the evaluation data by applying weightings (K1 to K6) between the first comparison presence time data and the second comparison presence time data.

3. A computer program comprising instructions to cause the evaluation device (100) according to claim 1 to execute the method of claim 2.

4. A computer-readable medium having stored thereon the computer program of claim 3.

**Patentansprüche**

1. Beurteilungsvorrichtung (100), die Folgendes aufweist:

eine Anzeige (101S), die konfiguriert ist, um Bilder anzuzeigen;

eine Blickpunktdetektionseinheit (214), die konfiguriert ist, um Positionsdaten eines Blickpunktes (P) einer Person zu detektieren, die die Anzeige (101) betrachtet;

eine Anzeigesteuervorrichtung (202), die konfiguriert ist, um nach der Darstellung einer Instruktionsinformation (I1 bis I4) für die Person ein spezielles Target- bzw. Zielobjekt (MA1 bis MA4) anzuzeigen, welches ein Bild ist und welches eine korrekte Antwort auf die Instruktionsinformation (I1 bis I4) ist und Vergleichstarget- bzw. Zielobjekte (MB1 bis MB6, MC1 bis MC12), die Bilder sind, und von dem spezifischen Zielobjekt (MA1 bis MA4) auf der Anzeige (101S) abweichen;

eine Bereichsfestlegungseinheit (216), die konfiguriert ist, um einen spezifischen Bereich (A) für das spezifische Zielobjekt (MA1 bis MA4) und Vergleichsbereiche (B1 bis B2, C1 bis C3) für die Vergleichszielobjekte (MB1 bis MB6, MC1 bis MC12) auf der Anzeige (101S) festzulegen;

eine Bestimmungseinheit (218), die konfiguriert ist, um basierend auf den Positionsdaten des Blickpunktes (P) zu bestimmen, ob der Blickpunkt (P) in sowohl dem spezifischen Bereich (A) als auch in den Vergleichsbereichen (B1 bis B2, C1 bis C3) vorhanden ist;

eine Arithmetikeinheit (220), die konfiguriert ist, um basierend auf einem Bestimmungsergebnis durch die Bestimmungseinheit (218) Blickpunktübergangsdaten zu berechnen; und

eine Beurteilungseinheit (224), die konfiguriert ist, um basierend auf den Blickpunktübergangsdaten Bewertungsdaten der Person zu erlangen; wobei

das Bild, auf welches die Person blicken soll, ein Polygonmuster ist;

die Anzeigesteuereinheit (202) weiter konfiguriert ist, um auf der Anzeige (101S) das spezifische Zielobjekt (MA1, MA2) anzuzeigen, welches das Polygonmuster ist, und welches die korrekte Antwort auf die Instruktionsinformation (I1 bis I4) ist, und die Vergleichszielobjekte (MB1 bis MB4, MC1 bis MC6), wobei jedes davon das Polygonmuster mit Ecken ist, deren Anzahl von jener des spezifischen Zielobjektes (MA1, MA2) abweicht;

die Vergleichszielobjekte zumindest ein erstes Vergleichszielobjekt (MB1 bis MB4) aufweisen, welches ein Polygonmuster mit Ecken ist, deren Anzahl von jener des spezifischen Zielobjektes (MA1, MA2) um 1 abweicht, und zumindest ein zweites Vergleichszielobjekt (MC1 bis MC6), welches ein Polygonmuster mit Ecken ist, deren Anzahl von jener des spezifischen Zielobjektes (MA1, MA2) um mehr als oder gleich 2 abweicht;

die Bereichsfestlegungseinheit (216) weiter konfiguriert ist, um als die Vergleichsbereiche zumindest einen ersten Vergleichsbereich (B1 bis B2) für das mindestens eine erste Vergleichszielobjekt (MB1 bis MB4) festzulegen, und mindestens einen zweiten Vergleichsbereich (C1 bis C3) für das mindestens eine zweite Vergleichszielobjekt (MC1 bis MC6) festzulegen;

die Zielpunktübergangsdaten weiter erste Vergleichsanwesenheitszeitdaten aufweisen, welche eine Anwesenheitszeit anzeigen, in welcher der Blickpunkt (P) in dem mindestens einen ersten Vergleichsbereich (B1 bis B2) anwesend ist, und zweite Vergleichsanwesenheitszeitdaten, die eine Anwesenheitszeit anzeigen, in welcher der Blickpunkt (P) in dem mindestens einen zweiten Vergleichsbereich (C1 bis C3) anwesend ist; und

die Beurteilungseinheit (224) weiter konfiguriert ist, um die Beurteilungsdaten zu erlangen durch Anwendung von Gewichtungen (K1 bis K6) zwischen den ersten Vergleichsanwesenheitszeitdaten und den zweiten Vergleichsanwesenheitszeitdaten.

2. Beurteilungsverfahren, welches Folgendes aufweist:

Anzeigen von Bildern auf einer Anzeige (101S);

Detektieren von Positionsdaten eines Blickpunktes (P) einer Person, welche die Anzeige (101S) betrachtet;

Anzeigen, und zwar nach dem Darstellen einer Instruktionsinformation (I1 bis I4) für die Person, eines spezifischen Zielobjektes (MA1 bis MA4), welches ein Bild ist und welches eine korrekte Antwort auf die Instruktionsinformation (I1 bis I4) ist, und von Vergleichszielobjekten (MB1 bis MB6, MC1 bis MC12), die Bilder sind, und von dem spezifischen Zielobjekt (MA1 bis MA4) abweichen, und zwar auf der Anzeige (101S);

Festlegen eines spezifischen Bereichs (A) auf der Anzeige (101S) für das spezifische Zielobjekt (MA1 bis MA4) und von Vergleichsbereichen (B1 bis B2, C1 bis C3) für die Vergleichszielobjekte (MB1 bis MB6, MC1 bis MC12) auf der Anzeige (101S);

Bestimmen, und zwar basierend auf den Positionsdaten des Blickpunktes (P), ob der Blickpunkt (P) in sowohl dem spezifischen Bereich (A) als auch in den Vergleichsbereichen (B1 bis B2, C1 bis C3) vorhanden ist;

Berechnen, und zwar basierend auf einem Bestimmungsergebnis, von Blickpunktübergangsdaten; und

Erlangen, und zwar basierend auf den Blickpunktübergangsdaten, von Beurteilungsdaten der Person; wobei das Bild, auf welches die Person blicken soll, ein Polygonmuster ist;

beim Anzeigen auf der Anzeige (101S) das spezifische Zielobjekt (MA1, MA2) angezeigt wird, welches das Polygonmuster ist, und welches die korrekte Antwort auf die Instruktionsinformationen (I1 bis I4) ist, und der Vergleichszielobjekte (MB1 bis MB4, MC1 bis MC6), wobei jedes davon das Polygonmuster ist, welches Ecken hat, deren Anzahl von jener des spezifischen Zielobjektes (MA1, MA2) abweicht;

die Vergleichszielobjekte zumindest ein erstes Vergleichszielobjekt (MB1 bis MB4) aufweisen, welches ein Polygonmuster mit Ecken ist, deren Anzahl von jener des spezifischen Zielobjektes (MA1, MA2) um 1 abweicht, und zumindest ein zweites Vergleichszielobjekt (MC1 bis MC6), welches ein Polygonmuster mit Ecken ist, deren Anzahl von jener des spezifischen Zielobjektes (MA1, MA2) um mehr als oder gleich 2 abweicht;

beim Festlegen das Festlegen von zumindest einem ersten Vergleichsbereich (B1 bis B2) für das mindestens eine erste Vergleichszielobjekt (MB1 bis MB4), und von mindestens einem zweiten Vergleichsbereich (C1 bis C3) für das mindestens eine zweite Vergleichszielobjekt (MC1 bis MC6) als die Vergleichsbereiche;

wobei die Blickpunktübergangsdaten weiter erste Vergleichsanwesenheitszeitdaten aufweisen, welche eine Anwesenheitszeit anzeigen, in welcher der Blickpunkt (P) in dem mindestens einen ersten Vergleichsbereich (B1 bis B2) anwesend ist, und zweite Vergleichsanwesenheitszeitdaten, die eine Anwesenheitszeit anzeigen, in welcher der Blickpunkt (P) in dem mindestens einen zweiten Vergleichsbereich (C1 bis C3) anwesend ist; und

beim Erlangen das Erlangen der Beurteilungsdaten durch Anwendung von Gewichtungen (K1 bis K6) zwischen den ersten Vergleichsanwesenheitszeitdaten und den zweiten Vergleichsanwesenheitszeitdaten.

3. Computerprogramm, welches Anweisungen aufweist, um die Bewertungsvorrichtung (100) nach Anspruch 1 zu veranlassen, das Verfahren nach Anspruch 2 auszuführen.

**4.** Computerlesbares Medium, auf dem das Computerprogramm nach Anspruch 3 gespeichert ist:

**Revendications**

**1.** Dispositif d'évaluation (100) comprenant :

un afficheur (101S) configuré pour afficher des images ;
une unité de détection de point de regard (214) configurée pour détecter des données de position d'un point de regard (P) d'un sujet qui observe l'afficheur (101S) ;
un contrôleur d'affichage (202) configuré pour afficher, après une présentation d'informations d'instructions (I1 à I4) au sujet, un objet cible spécifique (MA1 à MA4) qui est une image et qui est une réponse correcte aux informations d'instructions (I1 à I4) et à des objets cibles de comparaison (MB1 à MB6, MC1 à MC12) qui sont des images et qui diffèrent de l'objet cible spécifique (MA1 à MA4) sur l'afficheur (101S) ;
une unité de définition de zone (216) configurée pour définir une zone spécifique (A) pour l'objet cible spécifique (MA1 à MA4) et des zones de comparaison (B1 à B2, C1 à C3) pour les objets cibles de comparaison (MB1 à MB6, MC1 à MC12) sur l'afficheur (101S) ;
une unité de détermination (218) configurée pour déterminer, sur la base des données de position du point de regard (P), si le point de regard (P) est présent dans chacune parmi la zone spécifique (A) et les zones de comparaison (B1 à B2, C1 à C3) ;
une unité arithmétique (220) configurée pour calculer, sur la base d'un résultat de détermination par l'unité de détermination (218), des données de transition du point de regard ; et
une unité d'évaluation (224) configurée pour obtenir, sur la base des données de transition du point de regard, des données d'évaluation du sujet ; dans lequel
l'image qui doit être regardée par le sujet est un motif polygonal ;
l'unité de commande d'affichage (202) est en outre configurée pour afficher, sur l'afficheur (101S), l'objet cible spécifique (MA1, MA2) qui est le motif polygonal et qui est la réponse correcte aux informations d'instruction (I1 à I4) et aux objets cibles de comparaison (MB1 à MB4, MC1 à MC6) dont chacun est le motif polygonal ayant des coins, dont un nombre diffère de celui de l'objet cible spécifique (MA1, MA2) ;
les objets cibles de comparaison comportent au moins un premier objet cible de comparaison (MB1 à MB4) qui est un motif polygonal ayant des coins, dont le nombre diffère de celui de l'objet cible spécifique (MA1, MA2) de 1, et au moins un deuxième objet cible de comparaison (MC1 à MC6) qui est un motif polygonal ayant des coins, dont le nombre diffère de celui de l'objet cible spécifique (MA1, MA2) de 2 ou plus,
l'unité de définition de zone (216) est en outre configurée pour définir, en tant que zones de comparaison, au moins une première zone de comparaison (B1 à B2) pour l'au moins un premier objet cible de comparaison (MB1 à MB4) et au moins une deuxième zone de comparaison (C1 à C3) pour l'au moins un deuxième objet cible de comparaison (MC1 à MC6),
Les données de transition du point de regard comportent en outre des premières données de temps de présence de comparaison qui indiquent un temps de présence pendant lequel le point de regard (P) est présent dans l'au moins une première zone de comparaison (B1 à B2) et des deuxièmes données de temps de présence de comparaison qui indiquent un temps de présence pendant lequel le point de regard (P) est présent dans l'au moins une deuxième zone de comparaison (C1 à C3) ; et
l'unité d'évaluation (224) est en outre configurée pour obtenir les données d'évaluation en appliquant des pondérations (K1 à K6) entre les premières données de temps de présence de comparaison et les deuxièmes données de temps de présence de comparaison.

**2.** Procédé d'évaluation comprenant :

l'affichage d'images sur un afficheur (101S) ;
la détection de données de position d'un point de regard (P) d'un sujet qui observe l'afficheur (101S) ;
l'affichage, après une présentation d'informations d'instructions (I1 à I4) au sujet, d'un objet cible spécifique (MA1 à MA4) qui est une image et qui est une réponse correcte aux informations d'instructions (I1 à I4) et d'objets cibles de comparaison (MB1 à MB6, MC1 à MC12) qui sont des images et qui diffèrent de l'objet cible spécifique (MA1 à MA4) sur l'afficheur (101S) ;
la définition, sur l'afficheur (101S), d'une zone spécifique (A) pour l'objet cible spécifique (MA1 à MA4) et de zones de comparaison (B1 à B2, C1 à C3) pour les objets cibles de comparaison (MB1 à MB6, MC1 à MC12) sur l'afficheur (101S) ;
la détermination, sur la base des données de position du point de regard (P), de si le point de regard (P) est présent dans chacune parmi la zone spécifique (A) et les zones de comparaison (B1 à B2, C1 à C3) ;

le calcul, sur la base d'un résultat de la détermination, de données de transition du point de regard ; et

l'obtention, sur la base des données de transition du point de regard, de données d'évaluation du sujet dans lequel l'image qui doit être regardée par le sujet est un motif polygonal ;

au moment de l'affichage, l'affichage, sur l'afficheur (101S), de l'objet cible spécifique (MA1, MA2) qui est le motif polygonal et qui est la réponse correcte aux informations d'instruction (I1 à I4) et aux objets cibles de comparaison (MB1 à MB4, MC1 à MC6) dont chacun est le motif polygonal ayant des coins, dont un nombre diffère de celui de l'objet cible spécifique (MA1, MA2) ;

les objets cibles de comparaison comportent au moins un premier objet cible de comparaison (MB1 à MB4) qui est un motif polygonal ayant des coins, dont le nombre diffère de celui de l'objet cible spécifique (MA1, MA2) de 1, et au moins un deuxième objet cible de comparaison (MC1 à MC6) qui est un motif polygonal ayant des coins, dont le nombre diffère de celui de l'objet cible spécifique (MA1, MA2) de 2 ou plus,

au moment de la définition, la définition, en tant que zones de comparaison, d'au moins une première zone de comparaison (B1 à B2) pour l'au moins un premier objet cible de comparaison (MB1 à MB4) et d'au moins une deuxième zone de comparaison (C1 à C3) pour l'au moins un deuxième objet cible de comparaison (MC1 à MC6) ;

Les données de transition du point de regard comportent en outre des premières données de temps de présence de comparaison qui indiquent un temps de présence pendant lequel le point de regard (P) est présent dans l'au moins une première zone de comparaison (B1 à B2) et des deuxièmes données de temps de présence de comparaison qui indiquent un temps de présence pendant lequel le point de regard (P) est présent dans l'au moins une deuxième zone de comparaison (C1 à C3) ; et

au moment de l'obtention, l'obtention des données d'évaluation en appliquant des pondérations (KI à K6) entre les premières données de temps de présence de comparaison et les deuxièmes données de temps de présence de comparaison.

3.  Programme d'ordinateur comprenant des instructions qui amènent le dispositif d'évaluation (100) selon la revendication 1 à exécuter le procédé selon la revendication 2.

4.  Support lisible par ordinateur stockant un programme d'ordinateur selon la revendication 3.

# FIG.1

# FIG.2

# FIG.3

100

### 20

**202**
DISPLAY CONTROLLER

**204**
LIGHT SOURCE CONTROLLER

**206**
IMAGE DATA ACQUIRING UNIT

**208**
INPUT DATA ACQUIRING UNIT

**210**
POSITION DETECTING UNIT

**212**
CURVERTURE CENTER CALCULATING UNIT

**214**
GAZE POINT DETECTING UNIT

**216**
AREA SETTING UNIT

**218**
DETERMINATION UNIT

**220**
ARITHMETIC UNIT

**222**
STORAGE

**224**
EVALUATION UNIT

**226**
OUTPUT CONTROLLER

### 30

**302**
INPUT/ OUTPUT UNIT

### 40

**402**
DISPLAY DEVICE DRIVING UNIT

**404A**
FIRST CAMERA INPUT/ OUTPUT UNIT

**404B**
SECOND CAMERA INPUT/ OUTPUT UNIT

**406**
LIGHT SOURCE DRIVING UNIT

**101**
DISPLAY DEVICE

**102**

**102A**
FIRST CAMERA

**102B**
SECOND CAMERA

**103**

**103A**
FIRST LIGHT SOURCE

**103B**
SECOND LIGHT SOURCE

**50**
OUTPUT DEVICE

**60**
INPUT DEVICE

24

# FIG.4

# FIG.5

# FIG.6

# FIG.7

# FIG.8

101S

PLEASE GAZE AT
PENTAGON APPEARING NEXT.

I1

# FIG.9

MA1    A       MC3   C3        B2    MB2    101S

MB1   B1      MC2   C2        C1    MC1

P

# FIG.10

# FIG.11

## FIG.12

101S

PLEASE GAZE AT
HEXAGON APPEARING NEXT.

I2

## FIG.13

101S

B2  MB4  B1  MB3

MC6
C3

MC4
C1

C2  MC5  A  MA2

# FIG.14

101S

PLEASE GAZE AT
DICE WITH "4" PIPS.

I3

# FIG.15

MC7    C1        MC8    C2        B1    MB5      101S

MA3    A        MB6    B2        C3    MC9

# FIG.16

101S

PLEASE GAZE AT
AIRPLANE.

I4

# FIG.17

101S

C1

MC10

C2

MC11

P

A

MA4

C3

MC12

# FIG.18

```
                    START

                        │ S101
        ┌───────────────────────────┐
        │   START PLAYBACK OF VIDEO  │
        └───────────────────────────┘
                        │ S102
        ┌───────────────────────────┐
        │      WAITING TIME TO       │
        │   EVALUATION PURPOSE       │
        │      VIDEO PORTION         │
        └───────────────────────────┘
                        │ S103
        ┌───────────────────────────┐
        │      RESET TIMER T1        │
        └───────────────────────────┘
                        │ S104
        ┌───────────────────────────┐
        │   RESET COUNTER CNTA       │
        │   RESET COUNTER CNTB       │
        │   RESET COUNTER CNTC       │
        └───────────────────────────┘
                        │ S105
        ┌───────────────────────────┐
        │         FLAG=0             │
        └───────────────────────────┘
                        │ S106 TO S142
        ┌───────────────────────────┐
        │   PERFORM EVALUATION       │
        │        PROCESS             │
        └───────────────────────────┘
                        │ S143
              ╱─────────────────╲
        NO   ╱       HAS          ╲
        ◄────   TIMER T1 BEEN      
             ╲    COMPLETED?      ╱
              ╲─────────────────╱
                        │ YES  S144
        ┌───────────────────────────┐
        │   STOP PLAYBACK OF VIDEO   │
        └───────────────────────────┘
                        │ S145
        ┌───────────────────────────┐
        │   CALCULATE EVALUATION     │
        │         VALUE              │
        └───────────────────────────┘
                        │ S146
        ┌───────────────────────────┐
        │   OUTPUT EVALUATION        │
        │         VALUE              │
        └───────────────────────────┘
                        │
                      END
```

# FIG.19

**EP 3 884 873 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011083403 A
- JP 2017158866 A
- WO 2018216347 A1
- JP 2018140007 A